# EUROPEAN PATENT APPLICATION

(11) **EP 2 889 031 A1**
(43) Date of publication of application: **01.07.2015**
(21) Application number: 13825029.5
(22) Date of filing: 30.07.2013
(51) Int. Cl.: A61K 31/415, A61K 9/08, A61K 47/32, A61P 17/00, A61P 31/10

(54) **ANTITRICHOPHYTOSIS SOLUTION FOR EXTERNAL USE**

(30) Priority: 30.07.2012 WO PCT/JP2012/069306
(71) Applicant: Meiji Seika Pharma Co., Ltd., Tokyo 104-8002 (JP)
(72) Inventor: FURUTA, Yoshiko, Odawara-shi Kanagawa 250-0852 (JP); KOMATSU, Kazuki, Odawara-shi Kanagawa 250-0852 (JP); KAYA, Arpansiree, Odawara-shi Kanagawa 250-0852 (JP); TAKAHATA, Sho, Yokohama-shi Kanagawa 222-8567 (JP); TABATA, Yuji, Yokohama-shi Kanagawa 222-8567 (JP)
(74) Representative: Hoffmann Eitle
(86) International application number: PCT/JP2013/070525
(87) International publication number: WO 2014/021282

(57) **Abstract**

According to the present invention, a topical liquid agent having antifungal activity against dermatophytes, and further high nail permeability can be provided.

The topical liquid agent for nail and/or skin for prevention or treatment of dermatophytosis comprises a film-forming agent and a compound represented by the formula: 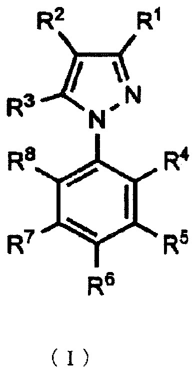 wherein R¹ represents a hydrogen atom, C₁₋₆ alkyl, or trifluoromethyl, R² represents a hydrogen atom, C₁₋₆ alkyl, halogen, -COO(C₁₋₆ alkyl), or (CH₂)₁₋₃COOR where R represents a hydrogen atom or C₁₋₆ alkyl, R³ represents a hydrogen atom, C₁₋₆ alkyl, amino, trifluoromethyl, or OR where R represents a hydrogen atom or C₁₋₆ alkyl, R⁴ represents a hydroxyl group, R⁵ represents a hydrogen atom, C₁₋₆ alkyl, a hydroxyl group, or halogen, R⁶ represents a hydrogen atom, C₁₋₆ alkyl, trifluoromethyl, halogen, amino, -NR^{a}R^{b}, nitro, hydroxy-C₁₋₆ alkyl, -CONR^{a}R^{b}, -COO(C₁₋₆ alkyl), -COOH, -(CH₂)₁₋₃COOR, or OR^{a} where R represents a hydrogen atom or C₁₋₆ alkyl, R^{a} and R^{b} may be the same or different from each other, and each represent a hydrogen atom, C₁₋₆ alkyl, or C₁₋₆ acyl, R⁷ represents a hydrogen atom, C₁₋₆ alkyl, -OR where R represents a hydrogen atom or C₁₋₆ alkyl, or halogen, R⁸ represents a hydrogen atom, C₁₋₆ alkyl, a hydroxyl group, amino, or nitro,
or a salt thereof.

## Description

### TECHNICAL FIELD

The present invention relates to a topical liquid agent for prevention or treatment of dermatophytosis comprising a novel 2-(1H-pyrazol-1-yl)phenol derivative or a pharmaceutically acceptable salt thereof suitable for an anti-dermatophytosis as an effective ingredient.

### BACKGROUND ART

Mycosis is a disease caused by a fungus which infects to human or animals. As representative mycoses in human, there have been known candidiasis due to *Candida,* cryptococcosis due to *Cryptococcus,* aspergillosis due to *Aspergillus,* zygomycosis due to *Zygomycetes,* dermatophytosis (tinea) due to *Trichophyton,* and the like (pathogenic fungi and mycosis (NANZANDO Co., Ltd., Revised 2nd Edition), pp.42-45; Non-Patent Document 1).

*Trichophyton* which is one of pathogenic fungi of mycosis is dermatophyte which could be pathogenic fungi of tinea, and has the keratinolytic ability. According to this ability, it invades into a skin, nail or hair to cause tinea (Pathogenic fungi and mycosis (NANZANDO Co., Ltd., Revised 2nd Edition), pp.184-187; Non-Patent Document 2).

Tinea unguium is a disease of a nail caused by dermatophytes which are said to be 90% or more of the causative fungi of onychomycosis in Japan, and a disease accompanied by symptoms of becoming turbid of the nail plate, thickening, destruction, and deformation, etc. At present, it has been said that there is one patient in ten Japanese, and about 12 million patients in total. This is a disease frequently appeared in aged person, and a further increase in a number of patients will be concerned in the future. There is a report that a diabetic patient easily contracts the disease so that the possibility of causing serious complications is being pointed out.

As a treatment method of onychomycosis in Japan nowadays, only an oral medicine of an antifungal agent (itraconazole and terbinafine, etc.) has been approved. However, the antifungal agent for oral administration has been pointed out that it has drug interaction, liver damage, and side effect(s) due to long term administration. In addition, an aged person, diabetic patient, etc., having a high morbidity of tinea unguium often takes multiple types of drugs, so that oral administration of the antifung agent for the treatment of tinea unguium is difficult in many cases (Br. J. of Dermatol., vol.139 (4), p.665, 1998; Non-Patent Document 3).

As for an anti-dermatophytosis topical agent, to exert the medical effect of the anti-dermatophytosis topical agent, it is required to deliver the drug to an epidermal horny layer and a nail which are infected portions, with a high level of a medical concentration in addition to have the medicine itself a potent antifungal activity against dermatophytes. However, the topical antifungal agent to be used for usual skin mycosis cannot reach to the horny or inside of the nail, or to a nail matrix with a sufficient concentration, whereby a sufficient effect cannot be obtained.

In abroad, a topical nail lacquer of amorolfine or ciclopirox has been approved and used. However, their permeabilities into the nail are very low, so that it cannot be expected to deliver to nail matrix cells by further permeation.

Therefore, a topical liquid agent of dermatophytosis such as tinea unguium, etc., is required to have not only a potent antifungal activity against dermatophytes, but also high permeability to nail, but such a topical liquid agent has not yet been found out.

On the other hand, as a compound having a 2-(1H-pyrazol-1-yl)phenol backbone, 2-(3,5-dimethyl-1H-pyrazol-1-yl)phenol which has methyl groups at the 3-position and 5-position of the pyrazole ring has been known (Annual Report of Takeda Research Center (1963) 22, p.27; Non-Patent Document 4). These compounds are directed to prevent growth of *Mycobacterium tuberculosis* in human. However, in Non-Patent Document 4, there is no disclosure of the 2-(1H-pyrazol-1-yl)phenol compound other than 2-(3,5-dimethyl-1H-pyrazol-1-yl)phenol.

As the compound having a 2-(1H-pyrazol-1-yl)phenol backbone other than 2-(3,5-dimethyl-1H-pyrazol-1-yl)phenol, 2-(1H-pyrazol-1-yl)phenol, 2-(3,5-dimethyl-1H-pyrazol-1-yl)-1,4-benzenediol and 2-(4-chloro-3,5-dimethyl-1H-pyrazol-1-yl)-1,4-benzenediol have been known. These have been utilized for various kinds of chemical reactions, a starting material as a compound for electroluminescence devices (JP Patent No. 4284169: Patent Document 1), a photostabilizer (Spanish Patent Laid-Open No. 20158648A: Patent Document 2), etc. Also, in WO 2003/005999 (Patent Document 3), 2-(5-amino-3-tert-butyl-1H-pyrazol-1-yl)-5-methylphenol is disclosed.

However, in these prior art literatures, there is neither suggestion nor disclosure about its antifungal activity against dermatophytes of the compound having a 2-(1H-pyrazol-1-yl)phenol backbone.

### PRIOR ART LITERATURES

### PATENT DOCUMENTS

[Patent Document 1] JP Patent No. 4284169
[Patent Document 2] Spanish Patent Laid-Open No. 20158648A
[Patent Document 3] WO 2003/005999A

### NON-PATENT DOCUMENTS

[Non-Patent Document 1] Pathogenic fungi and mycosis (NANZANDO Co., Ltd., Revised 2nd Edition), pp.42-45
[Non-Patent Document 2] Pathogenic fungi and mycosis (NANZANDO Co., Ltd., Revised 2nd Edition), pp.184-187
[Non-Patent Document 3] Br. J. of Dermatol., vol.139 (4), p.665, 1998
[Non-Patent Document 4] Annual Report of Takeda Research Center (1963) 22, p.27

### DISCLOSURE OF THE INVENTION

### PROBLEMS TO BE SOLVED BY THE INVENTION

As mentioned above, a topical liquid agent of superficial mycosis such as tinea unguium is required to have not only antifungal activity, but also high permeability to nail whereby development of such a topical liquid agent has been earnestly desired. Accordingly, an object of the present invention is to provide a topical liquid agent for nail and/or skin having an antifungal activity against dermatophytes, and having higher nail permeability.

### MEANS FOR SOLVING THE PROBLEMS

The present inventors have found that a topical liquid agent containing a compound having a 2-(1H-pyrazol-1-yl)phenol backbone represented by the following formula (I) or a salt thereof and a polymer is applied to nail and/or skin, then, it forms a film, so that it has high nail permeability, high storage stability, high antifungal activity against dermatophytes, and a precipitation-preventing effect of a drug, whereby they have accomplished the present invention based on these findings.

That is, the present invention relates to a topical liquid agent for nail and/or skin for prevention or treatment of dermatophytosis which comprises a film-forming agent and a compound represented by the formula: 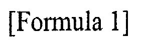 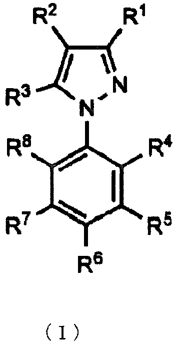 wherein
R¹ represents a hydrogen atom, C₁₋₆ alkyl, or trifluoromethyl,
R² represents a hydrogen atom, C₁₋₆ alkyl, halogen, -COO(C₁₋₆ alkyl), or (CH₂)₁₋₃COOR (R represents a hydrogen atom or C₁₋₆ alkyl),
R³ represents a hydrogen atom, C₁₋₆ alkyl, amino, trifluoromethyl, or OR (R represents a hydrogen atom or C₁₋₆ alkyl),
R⁴ represents a hydroxyl group,
R⁵ represents a hydrogen atom, C₁₋₆ alkyl, a hydroxyl group, or halogen,
R⁶ represents a hydrogen atom, C₁₋₆ alkyl, trifluoromethyl, halogen, amino, -NR^{a}R^{b}, nitro, hydroxy-C₁₋₆ alkyl, -CONR^{a}R^{b}, -COO(C₁₋₆ alkyl), -COOH, -(CH₂)₁₋₃COOR, or OR^{a} (R represents a hydrogen atom or C₁₋₆ alkyl, R^{a} and R^{b} may be the same or different from each other, and each represent a hydrogen atom, C₁₋₆ alkyl, or C₁₋₆ acyl),
R⁷ represents a hydrogen atom, C₁₋₆ alkyl, -OR (R represents a hydrogen atom or C₁₋₆ alkyl), or halogen, and
R⁸ represents a hydrogen atom, C₁₋₆ alkyl, a hydroxyl group, amino, or nitro,
or a salt thereof.

### EFFECTS OF THE INVENTION

The topical liquid agent of the present invention shows not only potent antifungal activity against dermatophytes, but also high permeability to a nail, so that it is useful as a topical liquid agent for nail and/or skin for prevention or treatment of dermatophytosis, particularly tinea unguium.

### BEST MODE FOR CARRYING OUT THE INVENTION

The terms "C₁₋₆ alkyl", and "C₁₋₆ alkyl" as a part of the group to be used in the present specification mean an alkyl group having 1 to 6 carbon atoms. The alkyl group may be either a linear, branched or cyclic one. There may be mentioned, for example, a C₁₋₆ alkyl group such as methyl, ethyl, propyl, isopropyl, butyl, isobutyl, sec-butyl, tert-butyl, pentyl, hexyl, etc., and a C₃₋₆ cycloalkyl group such as cyclopropyl, cyclobutyl, cyclopentyl and cyclohexyl.

The terms "C₁₋₆ acyl", and "C₁₋₆ acyl" as a part of the group to be used in the present specification mean an acyl group having 1 to 6 carbon atoms. The acyl group may be either a linear, branched or cyclic one. There may be mentioned, for example, formyl, acetyl, propionyl, butyryl, isobutyryl, etc., and preferably, formyl, acetyl, propionyl, butyryl, etc.

The term "halogen" used in the present specification means fluorine, chlorine, bromine, iodine, etc.

The terms of a group "-OR where R represents a hydrogen atom or C₁₋₆ alkyl" or a part of the group to be used in the present specification means a hydroxyl group, or C₁₋₆ alkyloxy. Here, "C₁₋₆ alkyl" is the same as the above-mentioned "C₁₋₆ alkyl".

In "-OR^{a}" as a group or a part of the group to be used in the present specification, R^{a} represents a hydrogen atom, C₁₋₆ alkyl, or C₁₋₆ acyl, and "-OR^{a}" represents a hydroxyl group, C₁₋₆ alkyloxy, or C₁₋₆ acyloxy. Here, "C₁₋₆ alkyl" is the same as the above-mentioned "C₁₋₆ alkyl".

With regard to Compound of formula (I) or a salt thereof and a solvate thereof

As mentioned above, the present invention relates to a topical liquid agent for nail and/or skin for prevention or treatment of dermatophytosis which comprises a film-forming agent and a compound represented by the formula: 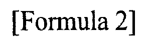 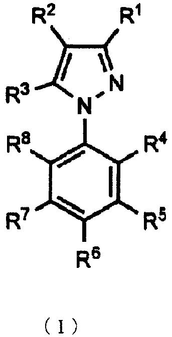 or a salt thereof.

In the formula (I), R¹ represents a hydrogen atom, C₁₋₆ alkyl, trifluoromethyl, preferably a hydrogen atom, or C₁₋₆ alkyl, and more preferably C₁₋₆ alkyl. R¹ specifically includes a hydrogen atom, methyl, ethyl, etc.

In the formula (I), R² represents a hydrogen atom, C₁₋₆ alkyl, halogen, -COO(C₁₋₆ alkyl), or -(CH₂)₁₋₃COOR (R represents a hydrogen atom or C₁₋₆ alkyl), preferably a hydrogen atom, C₁₋₆ alkyl, or halogen. R² specifically includes a hydrogen atom, methyl, ethyl, propyl, butyl, a chlorine atom, or 2-carboxyethyl, etc.

In the formula (I), R³ represents a hydrogen atom, C₁₋₆ alkyl, amino, trifluoromethyl, -OR (R represents a hydrogen atom or C₁₋₆ alkyl), preferably a hydrogen atom, or C₁₋₆ alkyl, more preferably C₁₋₆ alkyl. R³ specifically includes methyl, or ethyl.

In the formula (I), R⁴ represents a hydroxyl group.

In the formula (I), R⁵ represents a hydrogen atom, C₁₋₆ alkyl, a hydroxyl group, or halogen, preferably a hydrogen atom.

In the formula (I), R⁶ represents a hydrogen atom, C₁₋₆ alkyl, trifluoromethyl, halogen, amino, -NR^{a}R^{b}, nitro, hydroxy-C₁₋₆ alkyl, -CONR^{a}R^{b}, -COO(C₁₋₆ alkyl), -COOH, -(CH₂)₁₋₃COOR, or OR^{a} (R represents a hydrogen atom or C₁₋₆ alkyl, R^{a} and R^{b} may be the same or different from each other, and each represent a hydrogen atom, C₁₋₆ alkyl, or C₁₋₆ acyl), preferably a hydrogen atom, C₁₋₆ alkyl, trifluoromethyl, halogen, amino, -NR^{a}R^{b}, nitro, hydroxy-C₁₋₆ alkyl, -CONR^{a}R^{b}, -COOH, or OR^{a} (R^{a} and R^{b} may be the same or different from each other, and each represent a hydrogen atom, C₁₋₆ alkyl, or C₁₋₆ acyl), more preferably a hydrogen atom, C₁₋₆ alkyl, halogen, or OR^{a} (R^{a} represents a hydrogen atom, C₁₋₆ alkyl, or C₁₋₆ acyl). R⁶ specifically includes a hydrogen atom, methyl, ethyl, methoxy, a chlorine atom, a bromine atom, a fluorine atom, a hydroxyl group, acetoxy, amino, nitro, carboxyl, hydroxymethyl, trifluoromethyl, methylamino, carbamoyl, or N,N-dimethylcarbamoyl.

R⁷ represents a hydrogen atom, C₁₋₆ alkyl, -OR (R represents a hydrogen atom or C₁₋₆ alkyl), or halogen, preferably a hydrogen atom.

R⁸ represents a hydrogen atom, C₁₋₆ alkyl, a hydroxyl group, amino, or nitro, preferably a hydrogen atom, C₁₋₆ alkyl, amino, or nitro, more preferably a hydrogen atom. R⁸ specifically includes a hydrogen atom, methyl, nitro, or amino.

In the topical liquid agent for nail and/or skin for prevention or treatment of dermatophytosis of the present invention, a preferred compound of the formula (I) is a compound wherein
R¹ is a hydrogen atomor C₁₋₆ alkyl,
R² is a hydrogen atom, C₁₋₆ alkyl, halogen, or -(CH₂)₁₋₃COOR (R represents a hydrogen atom or C₁₋₆ alkyl),
R³ is a hydrogen atom, or C₁₋₆ alkyl,
R⁴ is a hydroxyl group,
R⁵ is a hydrogen atom,
R⁶ is a hydrogen atom, C₁₋₆ alkyl, trifluoromethyl, halogen, amino, -NR^{a}R^{b}, nitro, hydroxy-C₁₋₆ alkyl, -CONR^{a}R^{b}, -COOH, or OR^{a} (R^{a} and R^{b} may be the same or different from each other, and each represent a hydrogen atom, C₁₋₆ alkyl, or C₁₋₆ acyl),
R⁷ is a hydrogen atom, and
R⁸ is a hydrogen atom, C₁₋₆ alkyl, amino, or nitro (corresponding to Claim 2).

In the topical liquid agent for nail and/or skin for prevention or treatment of dermatophytosis of the present invention, a more preferred compound of the formula (I) is a compound wherein
R¹ is C₁₋₆ alkyl,
R² is a hydrogen atom, C₁₋₆ alkyl, or halogen,
R³ is C₁₋₆ alkyl,
R⁴ is a hydroxyl group,
R⁵ is a hydrogen atom,
R⁶ is a hydrogen atom, C₁₋₆ alkyl, halogen, or OR^{a} (R^{a} represents a hydrogen atom, C₁₋₆ alkyl, or C₁₋₆ acyl),
R⁷ is a hydrogen atom, and
R⁸ is a hydrogen atom (corresponding to Claim 3).

In the topical liquid agent for nail and/or skin for prevention or treatment of dermatophytosis of the present invention, a further preferred compound of the formula (I) is a compound wherein R¹ is C₁₋₆ alkyl, R² is a hydrogen atom, R³ is C₁₋₆ alkyl, R⁴ is a hydroxyl group, R⁵ is a hydrogen atom, R⁶ is C₁₋₆ alkyl, R⁷ is a hydrogen atom, and R⁸ is a hydrogen atom (corresponding to Claim 4).

As the compound of the formula (I) to be contained in the topical liquid agent for nail and/or skin for prevention or treatment of dermatophytosis of the present invention, the following compounds may be mentioned.
Preparation example 1: 2-(3,5-dimethyl-1H-pyrazol-1-yl)phenol
Preparation example 2: 2-(3,5-dimethyl-1H-pyrazol-1-yl)-4-fluorophenol
Preparation example 3: 2-(1H-pyrazol-1-yl)phenol
Preparation example 4: 2-(5-hydroxy-3-methyl-1H-pyrazol-1-yl)phenol
Preparation example 5: 2-(5-methyl-3-trifluoromethyl-1H-pyrazol-1-yl)phenol
Preparation example 6: 2-(3,5-bistrifluoromethyl-1H-pyrazol-1-yl)phenol
Preparation example 7: 2-(3-methyl-1H-pyrazol-1-yl)phenol
Preparation example 8: 2-(5-methyl-1H-pyrazol-1-yl)phenol
Preparation example 9: 2-(3,4,5-trimethyl-1H-pyrazol-1-yl)phenol
Preparation example 10: 2-(5-amino-3-tert-butyl-1H-pyrazol-1-yl)phenol
Preparation example 11: 4-chloro-2-(3,5-dimethyl-1H-pyrazol-1-yl)phenol
Preparation example 12: 2-chloro-6-(3,5-dimethyl-1H-pyrazol-1-yl)phenol
Preparation example 13: 2-(4-chloro-3,5-dimethyl-1H-pyrazol-1-yl)phenol
Preparation example 14: 2-(3,5-diethyl-1H-pyrazol-1-yl)phenol
Preparation example 15: 3-(3,5-dimethyl-1H-pyrazol-1-yl)benzene-1,2-diol
Preparation example 16: 2-(3,5-dimethyl-1H-pyrazol-1-yl)benzene-1,4-diol
Preparation example 17: 2-(4-ethyl-3,5-dimethyl-1H-pyrazol-1-yl)phenol
Preparation example 18: 5-fluoro-2-(3,4,5,-trimethyl-1H-pyrazol-1-yl)phenol
Preparation example 19: 2-(4-chloro-3,5-dimethyl-1H-pyrazol-1-yl)benzene-1,4-diol
Preparation example 20: 4-fluoro-2-(3,4,5,-trimethyl-1H-pyrazol-1-yl)phenol
Preparation example 21: 2-(4-chloro-3,5-dimethyl-1H-pyrazol-1-yl)-5-fluorophenol
Preparation example 22: ethyl
1-(2-hydroxyphenyl)-3,5-dimethyl-1H-pyrazol-4-carboxylate
Preparation example 23: methyl 3-(1-(2-hydroxyphenyl)-3,5-dimethyl-1H-pyrazol-4-yl)propanoate
Preparation example 24: 2-(4-butyl-3,5-dimethyl-1H-pyrazol-1-yl)phenol
Preparation example 25: 2-(3,5-dimethyl-1H-pyrazol-1-yl)-5-fluorophenol
Preparation example 26: 5-chloro-2-(3,5-dimethyl-1H-pyrazol-1-yl)phenol
Preparation example 27: 2-(3,5-dimethyl-1H-pyrazol-1-yl)-3-nitrophenol
Preparation example 28: 2-(3,5-dimethyl-1H-pyrazol-1-yl)-5-nitrophenol
Preparation example 29: 3-(1-(2-hydroxyphenyl)-3,5-dimethyl-1H-pyrazol-4-yl)propionic acid
Preparation example 30: 5-chloro-2-(3,4,5-trimethyl-1H-pyrazol-1-yl)phenol
Preparation example 31: 5-amino-2-(3,5-dimethyl-1H-pyrazol-1-yl)phenol
Preparation example 32: 5-nitro-2-(3,4,5-trimethyl-1H-pyrazol-1-yl)phenol
Preparation example 33: 4-(3,5-dimethyl-1H-pyrazol-1-yl)benzene-1,3-diol
Preparation example 34: 5-amino-2-(3,4,5-trimethyl-1H-pyrazol-1-yl)phenol
Preparation example 35: methyl 4-(3,5-dimethyl-1H-pyrazol-1-yl)-3-hydroxybenzenecarboxylate
Preparation example 36: 3-amino-2-(3,5-dimethyl-1H-pyrazol-1-yl)phenol
Preparation example 37: 4-(3,5-dimethyl-1H-pyrazol-1-yl)-3-hydroxybenzenecarboxylic acid
Preparation example 38: 4-(3,5-dimethyl-1H-pyrazol-1-yl)-3-hydroxy-N,N-dimethylbenzamide
Preparation example 39: 4-(3,5-dimethyl-1H-pyrazol-1-yl)-3-hydroxybenzamide
Preparation example 40: 3-hydroxy-4-(3,4,5-trimethyl-1H-pyrazol-1-yl)benzenecarboxylic acid
Preparation example 41: 3-hydroxy-4-(3,4,5-trimethyl-1H-pyrazol-1-yl)benzamide Preparation example 42: 4-(4-chloro-3,5-dimethyl-1H-pyrazol-1-yl)-3-hydroxybenzamide
Preparation example 43: 2-(3,5-dimethyl-1H-pyrazol-1-yl)benzene-1,3-diol
Preparation example 44: 2-(3,5-dimethyl-1H-pyrazol-1-yl)-5-methylphenol
Preparation example 45: 2-(3,5-dimethyl-1H-pyrazol-1-yl)-5-methoxyphenol
Preparation example 46: 2-(3,5-dimethyl-1H-pyrazol-1-yl)-3-methylphenol
Preparation example 47: 2-(3,5-dimethyl-1H-pyrazol-1-yl)-5-hydroxymethylphenol
Preparation example 48: 2-(3,5-dimethyl-1H-pyrazol-1-yl)-5-methylaminophenol
Preparation example 49: 2-(3,5-dimethyl-1H-pyrazol-1-yl)-4-methylphenol
Preparation example 50: 2-(3,5-dimethyl-1H-pyrazol-1-yl)-5-trifluoromethylphenol
Preparation example 51: 2-(3,5-dimethyl-1H-pyrazol-1-yl)-6-methylphenol
Preparation example 52: 2-(3,5-dimethyl-1H-pyrazol-1-yl)-5-ethylphenol
Preparation example 53: 2-(4-fluoro-3,5-dimethyl-1H-pyrazol-1-yl)phenol
Preparation example 54: 5-bromo-2-(3,5-dimethyl-1H-pyrazol-1-yl)phenol
Preparation example 55: 5-bromo-2-(4-chloro-3,5-dimethyl-1H-pyrazol-1-yl)phenol
Preparation example 56: 5-bromo-2-(3,4,5-trimethyl-1H-pyrazol-1-yl)phenol
Preparation example 57: 4-(3,5-dimethyl-1H-pyrazol-1-yl)-3-hydroxyphenyl acetate
Preparation example 58: 4-(4-chloro-3,5-dimethyl-1H-pyrazol-1-yl)-3-hydroxyphenyl acetate
Preparation example 59: 3-hydroxy-4-(3,4,5-trimethyl-1H-pyrazol-1-yl)phenyl acetate
Preparation example 60: 2-(3,5-dimethyl-1H-pyrazol-1-yl)-4-methoxy-5-methylphenol
Preparation example 61: 4-chloro-2-(3,5-dimethyl-1H-pyrazol-1-yl)-5-methylphenol
Preparation example 62: 2-(3,5-dimethyl-1H-pyrazol-1-yl)-4,5-dimethylphenol
Preparation example 63: 4-(4-chloro-3,5-dimethyl-1H-pyrazol-1-yl)-benzene-1,3-diol

Preferably mentioned is 2-(3,5-dimethyl-1H-pyrazol-1-yl)-5-methylphenol.

The compound of the formula (I) to be contained in the topical liquid agent for nail and/or skin for prevention or treatment of dermatophytosis of the present invention can be also used as a salt. As such a salt, there may be used a pharmaceutically acceptable optional salt, and may be specifically mentioned a pharmaceutically acceptable salt derived from an inorganic acid, an organic acid or a base. The "pharmaceutically acceptable salt" is known in this field of the art. For example, S. M. Berge et al. describe pharmaceutically acceptable salts in Journal of Pharmaceutical Sciences, 66, p.1 et seq. (1977) in detail. Representative acid addition salts may include an inorganic acid salt such as hydrochloride, sulfate, nitrate, hydrobromide, hydroiodide, phosphate, etc.; an organic carboxylic acid salt such as acetate, trifluoroacetate, lactate, citrate, oxalate, succinate, glutarate, malate, tartrate, fumarate, mandelate, maleate, benzoate, nicotinate, phthalate, etc.; an organic sulfonate such as methanesulfonate, ethanesulfonate, 2-hydroxyethanesulfonate, benzenesulfonate, p-toluenesulfonate, 2-naphthalenesulfonate, camphor sulfonate, etc.; an acidic amino acid salt such as aspartate, glutamate, etc., but the invention is not limited to these. The acid addition salt preferably includes a salt with an inorganic acid such as hydrochloric acid, hydrobromic acid, sulfuric acid and phosphoric acid; and a salt with an organic acid such as oxalic acid, maleic acid, methanesulfonic acid, p-toluenesulfonic acid and citric acid, more preferably a salt with hydrochloric acid, hydrobromic acid, sulfuric acid and methanesulfonic acid.

The base addition salt can be prepared by reacting a carboxylic acid or a phenolic-hydroxyl group-containing portion with a suitable base during the final isolation or purification process of the present invention compound to obtain the objective material at that time. The pharmaceutically acceptable salt may include an alkali metal salt such as lithium salt, sodium salt, potassium salt, etc.; an alkaline earth metl salt such as calcium salt, magnesium salt, etc.; aluminum salt, ammonium salt, and further an organic base salt such as methylamine salt, dimethylamine salt, ethylamine salt, diethylamine salt, trimethylamine salt, triethylamine salt, tetramethylammonium salt, tetraethylammonium salt, pyridine salt, picoline salt, ethanolamine salt, diethanolamine salt, triethanolamine salt, trishydroxymethylaminomethane salt, piperidine salt, piperazine salt, dicyclohexylamine salt, N,N-dibenzylethylenediamine salt, etc.; a basic amino acid salt such as arginine salt, lysine salt, ornithine salt, etc., but the invention is not limited to these. The base addition salt preferably includes an addition salt with sodium, potassium, calcium, ethanolamine and trishydroxymethylaminomethane, more preferably an addition salt with sodium, potassium and trishydroxymethylaminomethane.

Further, the compound of the formula (I) contained in the topical liquid agent for nail and/or skin for prevention or treatment of dermatophytosis of the present invention can be also used in the form of a solvate. The term "solvate" used in the present specification means complexes with various stoichiometry formed by a solute (the compound of the formula (I) or a salt thereof in the present invention) and a solvent. The solvent for the purpose of the present invention is preferably a solvent which does not interfere the biological activities of the above-mentioned solute and is preferably a pharmaceutically acceptable material. The suitable solvent for the solvate may be exemplified by water, methanol, ethanol, ethylene glycol, propylene glycol, ethyl acetate and butyl acetate, and the present invention is not limited by these. As a solvent for the solvate, water, ethanol and ethyl acetate are preferably mentioned.

### Preparation method of compound represented by the formula (I)

The 2-(1H-pyrazol-1-yl)phenol derivative represented by the formula (I) which is contained in the topical liquid agent of the present invention can be synthesized by an optional method, and, for example, it can be synthesized by the method shown in Scheme 1 or a method in accordance with the method. Incidentally, in Scheme 1, each symbol of the compound is the same as that mentioned above, P and P' each represent hydrogen or a suitable protective group, X represents a halogen or a suitable boronic acid group, and Y represents a dissociated ion of an acid used in the reaction. In the present specification, "suitable boronic acid" means boronic acid or a boronic acid ester.

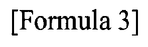

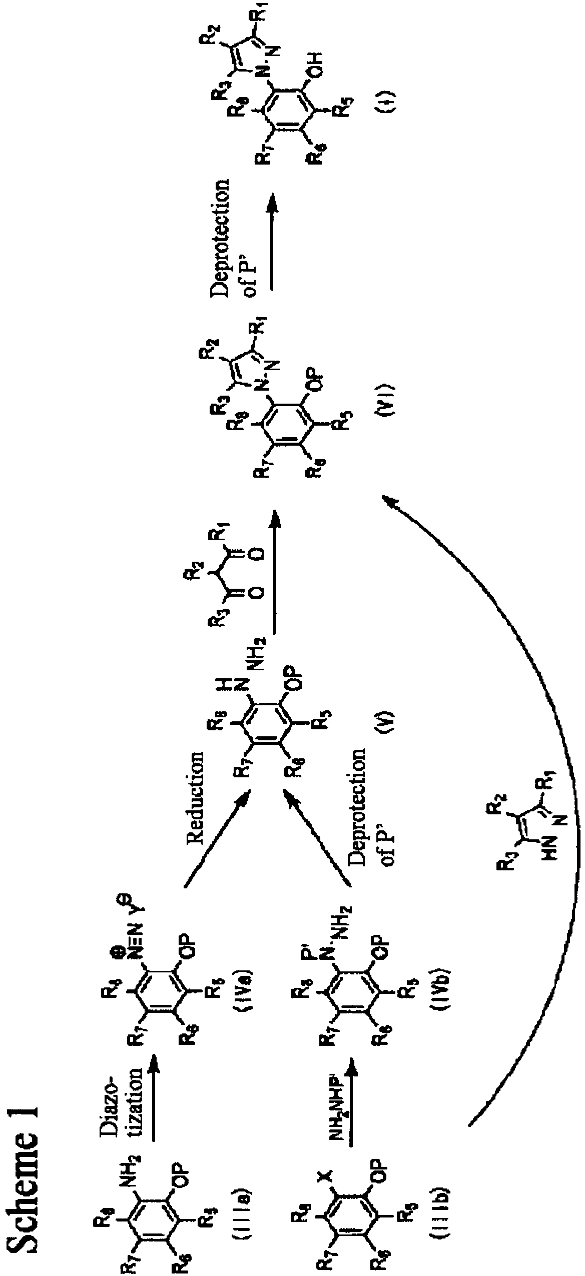

The method of obtaining a hydrazine derivative (V) or a salt thereof from a 2-hydroxyaniline derivative (IIIa) or a salt thereof via a diazonium compound (IVa) can be carried out in accordance with the method described in Organic Synthesis Collective Volume 1, pp.442-445, J. Org. Chem., vol.21, pp.394-399, 1956, WO 2007/083320, or US6852890.

The diazotization reaction can be carried out by using a nitrite such as potassium nitrite, calcium nitrite, silver nitrite, sodium nitrite, barium nitrite, etc.; nitrosylsulfuric acid or a nitrite ester such as ethyl nitrite, isoamyl nitrite, isobutyl nitrite, isopropyl nitrite, tert-butyl nitrite, n-butyl nitrite, n-propyl nitrite, etc., and preferably sodium nitrite, or a nitrite ester such as isoamyl nitrite, tert-butyl nitrite, etc. are mentioned.

When a protective group is used, any group may be used so long as it is inactive in the steps other than the deprotection, and as P and P', there may be mentioned, for example, an alkyl group such as a methyl group, an isopropyl group, an allyl group, a tert-butyl group, a methoxymethyl group, a methylthiomethyl group, a benzyl group and a 9-anthrylmethyl group, an acyl group such as a pivaloyl group and a benzoyl group, or a sulfonyl group such as a p-toluenesulfonyl group and a methanesulfonyl group, and the present invention is not limited by these. Preferred protective group may be mentioned a methyl group, a p-toluenesulfonyl group and a methanesulfonyl group.

The amount of the reagent to be used in the diazotization reaction is preferably 1 to 10 equivalents, more preferably 1 to 3 equivalents based on the amount of the 2-hydroxyaniline derivative (IIIa).

When a nitrite is used in the above-mentioned diazotization reaction, water, or an organic solvent mixed with water in an optional ratio, for example, methanol, ethanol, 2-propanol, acetic acid, trifluoroacetic acid, tetrahydrofuran, 1,4-dioxane, dimethylformamide and dimethyl sulfoxide may be used. In addition, these solvents may be used in combination of two or more. Preferably used are water, a water-methanol mixed solution, and a water-methanol-acetic acid mixed solution. Further, the diazotization reaction is carried out under acidic conditions to ensure solubility of the aniline derivative used as a substrate and to generate nitric acid in the reaction system. The acid to be used may include hydrochloric acid, hydrobromic acid, sulfuric acid, nitric acid, acetic acid, trifluoroacetic acid and phosphoric acid. Preferably used are hydrochloric acid, acetic acid and trifluoroacetic acid. Moreover, these acids may be also used as a solvent.

When a nitrite ester is used in the above-mentioned diazotization reaction, there may be used an alcohol such as methanol, ethanol, methoxyethanol, ethoxy ethanol, 1-propanol, 2-propanol, 1-butanol, 2-butanol and 2-methyl-2-propanol, an ether solvent such as diethyl ether, diisopropyl ether, tetrahydrofuran, methyl-tert-butyl ether, diphenyl ether and 1,4-dioxane, an acetic acid ester such as methyl acetate, ethyl acetate, propyl acetate and butyl acetate, an aprotic solvent such as N,N-dimethylform amide, N,N-dimethylacetamide, N-methylpyrrolidone and dimethyl sulfoxide, and a halogenated solvent such as dichloromethane, chloroform and 1,2-dichloroethane. In addition, these solvents may be used in combination of two or more. Preferably used are methanol, ethanol and a mixed solution of ethanol-diethyl ether.

The temperature of the above-mentioned diazotization reaction is in either of the case where a nitrite is used or the case where a nitrite ester is used both preferably -50°C to 50°C, more preferably between -30°C and 10°C, further preferably between -10°C and 5°C.

The above-mentioned diazotization reaction may vary depending on the substrate and the reaction conditions, and completes within 5 minutes to 12 hours, generally within 3 hours in many cases.

The concentration of the substrate in the solution of the above-mentioned diazotization reaction is not particularly limited and it can be carried out within the range of 0.1 mM to 10 M, preferably within the range of 1 mM to 1 M.

Reduction of the diazonium compound (IVa) to the hydrazine derivative (V) can be carried out by using stannous chloride or a hydrate thereof, a sulfite, a hydrogen sulfite, a dithionite, or triphenylphosphine (Organic Synthesis Collective Volume 1, p.442-445, J.Org.Chem., vol.21, pp.394-399, 1956, WO2007/083320, US6852890, US2007/0105866, J.Am.Chem.Soc., vol.92, pp.853-859, 1970). Preferred is a method using stannous chloride, a dithionite, or a sulfite.

The above-mentioned reduction reaction can be carried out subsequently to the diazotization reaction. That is, without isolating diazonium compound which is generally unstable, a reducing reagent is added to the reaction solution, or the diazotization reaction solution is added to the solution of the reducing reagent whereby the hydrazine derivative (V) or a salt thereof can be synthesized.

The amount of the above-mentioned reducing agent is preferably 1 to 30 equivalents, more preferably 1 to 10 equivalents based on the amount of the corresponding diazonium compound.

The solvent to be used in the above-mentioned reduction may be the same as that used in the diazotization reaction, also, may be optionally added, and preferably the same as that used in the diazotization reaction.

The temperature of the above-mentioned reduction may vary depending on the kind of the reducing agent, and preferably -50°C to 120°C, and the reduction may be more preferably carried out between -10°C and 70°C, and further preferably between -10°C and 30°C.

The hydrazine derivative (V) or a salt thereof can be synthesized from the compound (IIIb) without through the diazonium compound (IVa). That is, the compound (IIIb) is reacted with a hydrazine or a hydrazine protected by P' in the presence of a suitable catalyst, or in the absence thereof to obtain the hydrazine derivative (V) or a salt thereof.

The phenylpyrazole derivative (VI) can be similarly synthesized by reacting the compound (IIIb) with a suitable pyrazole in the presence of a suitable catalyst, or in the absence thereof.

When a hydrazine to which the protective group P' has bonded is used, the protective group may be any group so long as it is inactive in the steps other than the deprotection. As the P', there may be mentioned, for example, an alkyloxycarbonyl group such as a methoxycarbonyl group, an ethoxycarbonyl group, a tert-butoxycarbonyl group and a benzyloxycarbonyl group, etc., an acyl group such as a pivaloyl group and a benzoyl group, or a sulfonyl group such as a p-toluenesulfonyl group and a methanesulfonyl group, and the present invention is not limited by these. As the preferred protective group, there may be mentioned a t-butoxycarbonyl group.

When X of the compound (IIIb) is halogen, the reaction can be carried out in accordance with the method disclosed in Organic Letters vol.3, pp.3803-3805, 2001, J.Org.Chem., vol.72, pp.6190-6199, 2007, or J.Org.Chem., vol.70, pp.5164-5173, 2005. When X of the compound (IIIb) is boronic acid, the reaction can be carried out in accordance with the method disclosed in Bioorg. Med. Chem. Lett., vol.18, pp.4438-4441, 2008.

The amount of the hydrazine or the hydrazine protected by P' to be used in the above-mentioned reaction, or an amount of the pyrazole is preferably 1 to 30 equivalents, more preferably 1 to 5 equivalents based on the amount of the compound (IIIb).

The solvent suitable for the above-mentioned reaction may vary depending on the substrate or the reaction conditions, and there may be mentioned an aprotic solvent such as N,N-dimethylformamide, N,N-dimethylacetamide, N-methylpyrrolidone, dimethyl sulfoxide, sulfolane, acetonitrile and propionitrile, an ether solvent such as 1,4-dioxane, 1,2-dimethoxyethane and 1,2-diethoxyethane, and a halogenated solvent such as chloroform, 1,2-dichloroethane, 1,1,2,2-tetrachloroethane and 1,1,1-trichloroethane, and the present invention is not limited by these. In addition, these solvents may be used in combination of two or more. When the substrate is a liquid, it is possible to react the materials without using any solvent. The reaction is preferably carried out by using N,N-dimethylformamide, N-methylpyrrolidone, propionitrile or dimethyl sulfoxide, or without any solvent.

In the above-mentioned reaction, in addition to the salt of copper or palladium, a material in which a suitable ligand is coordinated to copper or palladium may be used with a catalytic amount or with a stoichiometric amount or more. At that time, it is preferred to also use an organic base such as 1,4-diazabicyclo[2,2,2]octane, 1,8-diazabicyclo[5,4,0]undecene, pyridine and N,N-dimethylaminopyridine, or an inorganic base such as potassium tert-butoxide, sodium tert-butoxide, or potassium carbonate, cesium carbonate and potassium phosphate. Pyridine, potassium carbonate, cesium carbonate, potassium phosphate, etc. are more preferred.

As a suitable ligand, there may be mentioned tributylphosphine, triphenylphosphine, N-methylglycine, N,N-dimethylglycine, 1,2-diaminocyclohexane, 1,10-phenanthroline derivative, 8-hydroxyquinoline, picolinic acid and 2,2'-bipyridine, and the present invention is not limited by these. N,N-dimethylglycine, 1,2-diaminocyclohexane or 8-hydroxyquinoline are more preferred.

When a small amount of water, polyethylene glycol, etc., is added, good results can be sometimes obtained in the above-mentioned reaction.

When X of the compound (IIIb) is boronic acid, good results can be sometimes obtained if air or oxygen is optionally blown into the reaction system.

The temperature of the above-mentioned reaction may vary depending on the kind of the substrate, presence or absence of the catalyst or the kind of the same, and is preferably 10°C to 200°C, more preferably 20°C to 150°C. At this time, when a microwave is irradiated, the reaction is sometimes accelerated.

The above-mentioned reaction may vary depending on the kind of the substrate, presence or absence of the catalyst or the kind of the same, and completes within 15 minutes to 96 hours, generally within 48 hours in many cases.

The concentration of the substrate in the above-mentioned reaction is not particularly limited, and the reaction is generally carried out with a concentration of 1 mM to neat (without solvent). It is preferably 10 mM to 10 M.

The deprotection of the compound (IVb) to the compound (V) or a salt thereof may be carried out by using a suitable method depending on the P' used in view of Green, Protective Groups in Organic Synthesis (5th), 1999, John Wieley & Sons. Specifically, when the P' is a tert-butoxycarbonyl group, an acid such as hydrochloric acid and trifluoroacetic acid is preferably used. At that time, when anisole or thioanisole is coexisted, good results can be sometimes obtained.

The obtained hydrazine derivative (V) or a salt thereof is reacted with 1,3-diketone or its chemical equivalent to synthesize a phenylpyrazole derivative (VI) in which a pyrazole ring is formed. Here, the chemical equivalent means a compound in which the carbonyl group is protected by an acetal group, which can be easily converted into a ketone group by an acid existing in the system during the pyrazole ring-forming reaction.

The amount of the 1,3-diketone or its chemical equivalent to be used in the above-mentioned reaction is preferably 1 to 20 equivalents, more preferably 1 to 5 equivalents based on the amount of the compound (V).

The solvent suitable for the above-mentioned reaction may vary depending on the substrate or the reaction conditions, and may include an alcohol such as methanol, ethanol, methoxyethanol, ethoxyethanol, 1-propanol, 2-propanol, 1-butanol, 2-butanol, 2-methyl-2-propanol, glycerol and 1,3-propanediol, an ether solvent such as diethyl ether, diisopropyl ether, tetrahydrofuran, methyl-tert-butyl ether, diphenyl ether, 1,4-dioxane, diethylene glycol dimethyl ether, 1,2-dimethoxyethane and 1,2-diethoxyethane, an acetic acid ester such as methyl acetate, ethyl acetate, propyl acetate and butyl acetate, an aprotic solvent such as N,N-dimethylformamide, N,N-dimethylacetamide, N-methylpyrrolidone, dimethyl sulfoxide, acetonitrile and propionitrile, and a halogenated solvent such as dichloromethane, chloroform, 1,2-dichloroethane, 1,1,2,2-tetrachloroethane and 1,1,1-trichloroethane, and the present invention is not limited by these. In addition, these solvents may be used in combination of two or more. It is preferred to use methanol, ethanol, 2-propanol, 1,2-dimethoxyethane and N,N-dimethylformamide.

When the hydrazine derivative (V) is used as a free material in the above-mentioned reaction, a suitable acid may be added in an amount of a catalytic amount or 1 equivalent or more.

As a suitable acid, there may be mentioned hydrochloric acid, hydrobromic acid, sulfuric acid, nitric acid, acetic acid, trifluoroacetic acid and phosphoric acid, preferably hydrochloric acid and trifluoroacetic acid.

The temperature of the above reaction may vary depending on the kind of the substrates, and is preferably at 10°C to 200°C, more preferably 40°C to 120°C.

The above-mentioned reaction may vary depending on the kind of the substrate, and completes within 15 minutes to 24 hours, generally within 12 hours in many cases.

The concentration of the substrate in the above-mentioned reaction is not particularly limited, and the reaction is generally carried out with a concentration of 0.1 mM to 1 M, preferably 10 mM to 1 M.

The obtained phenylpyrazole derivative (VI) can be led to the compound (I) of the present invention, by deprotection reaction of the protective group if necessary. The deprotection may be carried out by using a suitable method depending on the P used in view of Green, Protective Groups in Organic Synthesis (5th), 1999, John Wieley & Sons. When P is a methyl group, boron tribromide, aluminum chloride, etc., is preferably used, when P is a benzyl group, catalytic hydrogenation and reduction, etc., is preferably used, and when P is a p-toluenesulfonyl group, sodium hydroxide or potassium hydroxide, etc., is preferably used.

The compound (I) of the present invention may be further chemically modified to one or both of the benzene ring side chain and the pyrazole ring side chain by an organic chemical reaction generally used. For example, the compound having a carboxyl group may be subjected to esterification, amidation, or reduction to alcohol, the compound having an amino group may be subjected to alkylation, acylation, or carbamation reaction, which are the reactions easily conceived by those skilled in the art having knowledge in organic chemistry.

Also, the compound (I) of the present invention can be synthesized by using a 2-nitroaniline derivative (IIIc), or a nitrobenzene derivative (IIId) having a halogen or a boronic acid group at the 2-position as shown in Scheme 2. Incidentally, in Scheme 2, P' represents hydrogen or a suitable protective group, X represents halogen or a suitable boronic acid group, and Y represents a dissociated ion of an acid used in the reaction.
[Formula 4]

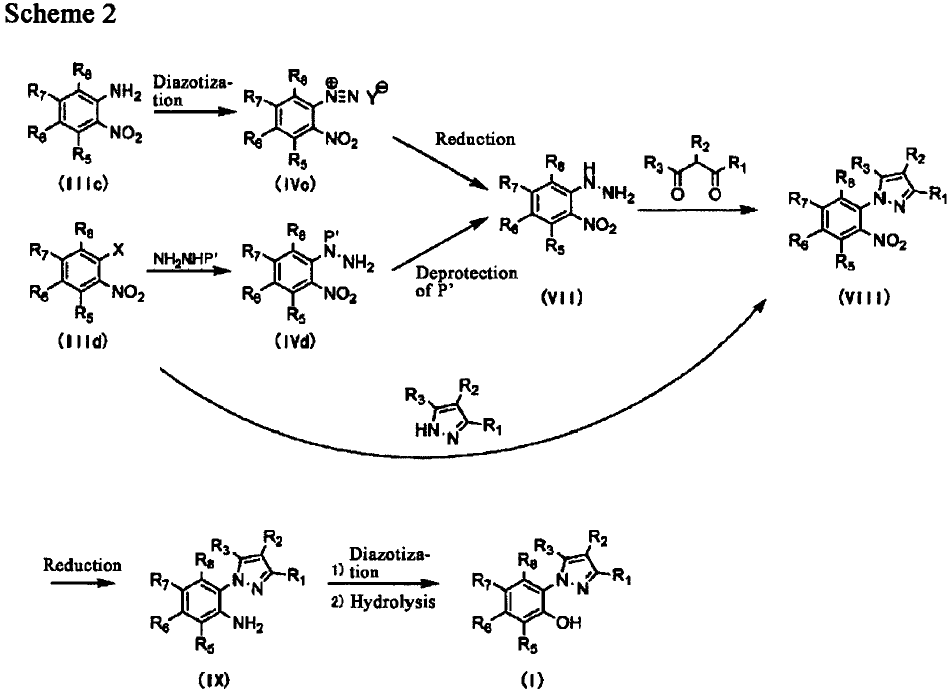

Diazotization of the 2-nitroaniline derivative (IIIc), reduction of the obtained diazonium compound, and cyclization of the pyrazole ring can be carried out in accordance with the preparation method of the above-mentioned reaction from (IIIa) to (V), and, from (V) to (VI).

The reaction from the nitrobenzene derivative (IIId) to the 2-nitrophenylpyrazole derivative (VIII) can be carried out in accordance with the preparation method of the above-mentioned reaction from (IIIb) to (VI). Or else, it can be synthesized in accordance with the method described in J. Org. Chem., vol.76, pp.654-660, 2011.

Reduction from the 2-nitrophenylpyrazole derivative (VIII) to the 2-amino-phenylpyrazole derivative (IX) can be carried out in view of Experimental Chemistry, fourth edition, pp.159 to 266, and utilizing the catalytic hydrogenation and reduction, in the presence of an acid, reduction by a metal, reduction by a metal hydride, and the like.

In the case of the catalytic hydrogenation and reduction, the reaction is carried out with a hydrogen pressure of 1 to 80 atm, preferablyl to 5 atm.

The solvent suitable for the above-mentioned reaction may vary depending on the substrate or the reaction conditions, and there may be mentioned an alcohol such as methanol, ethanol, methoxyethanol, ethoxyethanol, 1-propanol, 2-propanol, 1-butanol, 2-butanol and 2-methyl-2-propanol, an ether solvent such as diethyl ether, diisopropyl ether, tetrahydrofuran, methyl-t-butyl ether, diphenyl ether, diethylene glycol dimethyl ether and 1,4-dioxane, an acetic acid ester such as methyl acetate, ethyl acetate, propyl acetate and butyl acetate, and N,N-dimethylformamide, N,N-dimethylacetamide, N-methylpyrrolidone, dimethyl sulfoxide, glycerol, 1,3-propanediol, 1,2-dimethoxyethane, 1,2-diethoxyethane, water and acetic acid, and the present invention is not limited by these. In addition, these solvents may be used in combination of two or more. It is preferred to use methanol, ethanol, 2-propanol and 1,2-dimethoxyethane.

As the catalyst to be used in the catalytic hydrogenation and reduction, there may be mentioned a metal such as palladium, platinum, rhodium and nickel, and a complex thereof, these compounds or a salt thereof which are adsorbed to activated carbon, etc., and preferably palladium carbon and Raney nickel.

The temperature of the above-mentioned catalytic hydrogenation reduction may vary depending on the kind of the substrates and catalysts, and is preferably 0°C to 100°C, more preferably 10°C to 50°C.

The above-mentioned catalytic hydrogenation and reduction may vary depending on the kind of the substrate, and completes within 15 minutes to 24 hours, generally within 12 hours in many cases.

The concentration of the substrate in the above-mentioned catalytic hydrogenation and reduction is not particularly limited, and the reaction is generally carried out with a concentration of 0.1 mM to 1M, preferably 1 mM to 100 mM.

When a metal is used as a reducing agent, the metal to be used is iron, tin, zinc, etc., and it is necessary to add an acid in either of the cases. As the acid to be used there may be mentioned hydrochloric acid, sulfuric acid, acetic acid, trifluoroacetic acid and phosphoric acid, preferably hydrochloric acid. Moreover, these acids may be used also as a solvent.

The amount of the metal to be used in the above-mentioned reaction is preferably 1 to 100 equivalents, more preferably 3 to 15 equivalents based on the amount of the compound (VIII).

The solvent suitable for the above-mentioned reaction may vary depending on the substrate or the reaction conditions, and there may be mentioned water, acetic acid, an alcohol such as methanol, ethanol, methoxyethanol, ethoxyethanol, 1-propanol, 2-propanol, 1-butanol, 2-butanol and 2-methyl-2-propanol, an ether solvent such as diethyl ether, diisopropyl ether, tetrahydrofuran, diphenyl ether, diethylene glycol dimethyl ether and 1,4-dioxane, an acetic acid ester such as methyl acetate, ethyl acetate, propyl acetate and butyl acetate, and N,N-dimethylformamide, N,N-dimethylacetamide, N-methylpyrrolidone, dimethyl sulfoxide, glycerol, 1,3-propanediol, 1,2-dimethoxyethane and 1,2-diethoxyethane, and the present invention is not limited by these. In addition, these solvents may be used in combination of two or more. It is preferred to use hydrochloric acid, acetic acid, ethanol, 2-propanol and a mixture thereof.

The temperature of the above-mentioned reaction may vary depending on the kind of the substrates or conditions, and is preferably 0°C to 100°C, more preferably 20°C to 50°C.

The above-mentioned reaction may vary depending on the kind of the substrate and the conditions, and completes within 1 hour to 24 hours, generally within 12 hours in many cases.

The concentration of the substrate in the above-mentioned reaction is not particularly limited, and the reaction is generally carried out with a concentration of 0.1 mM to 1 M, preferably 1 mM to 100 mM.

In the case of the reduction reaction using a metal hydride, there may be used, as a reagent, lithium borohydride, sodium borohydride, potassium borohydride, zinc borohydride, lithium triethoxyborane and diisobutylaluminum hydride, preferably lithium borohydride or sodium borohydride. At that time, good results can be sometimes be obtained when stannous chloride, nickel(II) chloride, etc., is coexisted.

The amount of the reducing agent to be used in the above-mentioned reaction is preferably 1 to 50 equivalents, more preferably 1 to 5 equivalents based on the amount of the compound (VIII).

The solvent suitable for the above-mentioned reaction may vary depending on the kinds of the substrate or the reducing agent, and there may be mentioned an ether solvent such as diethyl ether, diisopropyl ether, tetrahydrofuran, diphenyl ether, 1,4-dioxane, diethylene glycol dimethyl ether, 1,2-dimethoxyethane and 1,2-diethoxyethane, and an alcohol such as methanol, ethanol, methoxyethanol, ethoxyethanol, 1-propanol, 2-propanol, 1-butanol, 2-butanol and 2-methyl-2-propanol, and the present invention is not limited by these. In addition, these solvents may be used in combination of two or more. It is preferred to use methanol and a mixture of methanol and diethylene glycol dimethyl ether.

The temperature of the above-mentioned reaction may vary depending on the kind of the substrates or conditions, and is preferably -80°C to 100°C, more preferably -20°C to 80°C.

The above-mentioned reaction may vary depending on the kind of the substrate and the conditions, and completes within 15 minutes to 24 hours, generally within 12 hours in many cases.

The concentration of the substrate in the above-mentioned reaction is not particularly limited, and the reaction is generally carried out with a concentration of 0.1 mM to 1 M, preferably 1 mM to 100 mM.

The obtained 2-aminophenylpyrazole derivative (IX) can be diazotized in accordance with the above-mentioned preparation method of from (IIIa) to (IVa) and Japanese Unexamined Patent Publication Hei 8-53401.

The reaction from the diazonium compound to (I) can be carried out in accordance with the method described in Japanese Unexamined Patent Publication Hei 8-188545 and Japanese Unexamined Patent Publication Hei 11-60528.

Hydrolysis of the above-mentioned diazonium salt can be carried out under acidic conditions and heating in water or in a solvent containing water.

The solvent suitable for the above-mentioned reaction may vary depending on the substrate or the reaction conditions, and there may be mentioned water, acetic acid, trifluoroacetic acid, an alcohol such as methanol, ethanol, methoxyethanol, ethoxyethanol, glycerol, 1,3-propanediol, 1-propanol, 2-propanol, 1-butanol, 2-butanol and 2-methyl-2-propanol, an ether solvent such as tetrahydrofuran, diethylene glycol dimethyl ether, 1,2-dimethoxyethane, 1,2-diethoxyethane and 1,4-dioxane, and an aprotic solvent such as N,N-dimethylformamide, N,N-dimethylacetamide, N-methylpyrrolidone and dimethyl sulfoxide, and the present invention is not limited by these. In addition, these solvents may be used in combination of two or more. It is preferred to use water, sulfuric acid, hydrochloric acid, trifluoroacetic acid, acetic acid, methanol, ethanol and a mixture of optional combination thereof.

The temperature of the above-mentioned reaction may vary depending on the kind of the substrates or conditions, and is preferably 20°C to 200°C, more preferably 50°C to 150°C.

The above-mentioned reaction may vary depending on the kind of the substrate and the conditions, and completes within 10 minutes to 24 hours, generally within 12 hours in many cases.

The concentration of the substrate in the above-mentioned reaction is not particularly limited, and the reaction is generally carried out with a concentration of 0.1 mM to 1 M, preferably 1 mM to 100 mM.

### With regard to film-forming agent

The topical liquid agent of a nail and/or skin for prevention or treatment of dermatophytosis of the present invention contains the compound represented by the above-mentioned formula (I) or a salt thereof in combination with the film-forming agent. Such a film-forming agent can be used any optional film-forming agent generally used for a topical liquid agent for a nail or a skin. Such a film-forming agent may contain a solvent and an additive. The additive may be mentioned, for example, a polymer, and the solvent may be mentioned an organic solvent, etc.

The polymer may be mentioned a water-soluble polymer and a water-insoluble polymer, etc.

The water-soluble polymer may be mentioned copolyvidone, povidone, hydroxypropyl cellulose, or, hypromellose, polyvinyl alcohol, etc., and the water-insoluble polymer may be mentioned an acrylic resin alkanolamine, or, aminoalkyl methacrylate copolymer RS, aminoalkyl methacrylate copolymer E, polyvinyl acetal diethylaminoacetate, etc.

The solvent may be mentioned a lower alcohol, ethyl acetate, butyl acetate, etc., and the lower alcohol may be mentioned methanol, ethanol, isopropanol, etc., and preferably mentioned is ethanol.

### (1) Water-soluble polymer-containing film-forming agent

The water-soluble polymer-containing film-forming agent may be mentioned, for example, copolyvidone (Plasdone S-630 available from Ashland Inc., or Kollidon VA64 available from BASF AG), povidone (Plasdone K-12, Plasdone K-17, Plasdone K-25, Plasdone K-29/32, Plasdone K-90, Plasdone K-90D all available from Ashland Inc.), hydroxypropyl cellulose (HPC-SSL, HPC-SL, HPC-L available from NIPPON SODA Co., Ltd.), hypromellose (TC-5E, TC-5R, TC-5M available from Shin-Etsu Chemical Co., Ltd.), polyvinyl alcohol (Gohsenol available from Nippon Synthetic Chemical Industry Co., Ltd.), etc.

### (2) Water-insoluble polymer-containing film-forming agent

The water-insoluble polymer-containing film-forming agent may be mentioned, for example, acrylic resin alkanolamine (PLAS CIZE L-53 series, PLAS CIZE L-9000 series both available from GOO CHEMICAL CO., LTD.), aminoalkyl methacrylate copolymer RS (EUDRAGIT RS, EUDRAGIT RL, etc., all available from EVONIK), aminoalkyl methacrylate copolymer E (EUDRAGIT E available from EVONIK), polyvinyl acetal diethylaminoacetate (AEA SANKYO available from Sankyo Co., Ltd.), etc.

Formulation ratio of the compound of the formula (I) or a salt thereof based on the whole amount of the liquid agent

A formulation ratio of the compound of the formula (I) or a salt thereof in the topical liquid agent for nail and/or skin of the present invention can be optionally determined depending on the kind of the compound. When the compound of the formula (I) or a salt thereof is formulated with a water-soluble polymer-containing film-forming agent or a water-insoluble film-forming agent, the compound of the formula (I) or a salt thereof is formulated in an amount of preferably 1 to 50% by mass, more preferably 1 to 30% by mass, further more preferably 1 to 20% by mass, particularly preferably 5 to 15% by mass into the whole liquid amount with a total mass standard.

### Other additives

In the topical liquid agent for nail and/or skin of the present invention, a preservative, a solubilizing agent, a permeation enhancer, an antioxidant, a viscosity-increasing agent, etc., which are generally used for a topical agent may be contained in addition to the above-mentioned film-forming agent.

As the preservative, there may be formulated, for example, organic preservatives (cationic activator, phenols, sorbate, salicylate, dehydroacetate and benzoate, etc.), and inorganic preservatives (antibacterial zeolite in which an antibacterial metal ion such as silver, copper, zinc, etc., is ion-exchanged with zeolite, etc.).

As the solubilizing agent, there may be mentioned a solubilizing agent including, for example, polyvalent alcohols (glycerine, sorbitol, ethylene glycol, propylene glycol, dipropylene glycol, 1,3-butylene glycol, 1,3-tetramethylene glycol, polyethylene glycol, etc.), phenols (thymol, safrole, isosafrole, eugenol, isoeugenol, etc.), higher alcohols (benzyl alcohol, oleyl alcohol, cetyl alcohol, stearyl alcohol, cetostearyl alcohol, octyldodecanol, etc.), ester type surfactants (sesquioleic acid sorbitane, polyoxyethylene hydrogenated castor oil, polyoxyl stearate, etc.), fatty acid esters (isopropyl myristate, octyldodecyl myristate, oleyl oleate, diethyl phthalate, dibutyl phthalate, etc.), (meth)acrylates (aminoalkyl methacrylate copolymer, etc.), and organic acids (lactic acid, citric acid, tartaric acid, maleic acid, malic acid, etc.).

As the permeation enhancer, there may be mentioned a permeation enhancer including, for example, an aliphatic acid, an aliphatic acid ester (isopropyl myristate, isopropyl palmitate, diisopropyl sebacate, diethyl sebacate, diisopropyl adipate, diethyl adipate, etc.), an aliphatic acid amide, an aliphatic alcohol, 2-(2-ethoxyethoxy)-ethanol, an ester of glycerol, glycerol monolaurate, propylene glycol, polyethylene glycol, an unsaturated polyglycolated glyceride, saturated polyglyceride, α-hydroxy acid, dimethyl sulfoxide, decylmethyl sulfoxide, pyrrolidones, salicylic acid, lactic acid, dimethylformamide, dimethylacetamide, sodium dodecyl sulfate, phospholipid, oleic acid, oleic acid/2-(2-ethoxyethoxy)ethanol, protease and N-acetylcysteine.

The antioxidant may be mentioned, for example, 1,3-butylene glycol, citric acid hydrate (monohydrate), tocopherol, tocopherol acetic acid ester, ascorbyl palmitate, butylhydroxyanisole (BHA), dibutylhydroxy toluene (BHT), propyl gallate, etc.

The viscosity-increasing agent for improving tactile sense may be mentioned, for example, silicone derivatives (cyclic silicone, dimethylpolysiloxane etc.), hydrophobized hydroxypropylmethylcellulose, poloxamer, etc. Thus, in one embodiment, the topical liquid agent for nail and/or skin of the present invention comprises a viscosity-increasing agent, preferably a silicone derivative, hydrophobized hydroxypropylmethylcellulose or poloxamer.

### Preparation method of topical liquid agent of the present invention

For preparing the topical liquid agent of the present invention, in a part of the solvent were dissolved successively the compound of the formula (I) or a salt thereof which is an active ingredient, and the film-forming agent, etc., and amounts thereof are adjusted with a solvent to obtain a preparation. Also, for preparing the topical liquid agent of the present invention, a method for preparing the usual topical liquid agent can be optionally used. In the following, the present invention will be explained by referring to Examples, but the present invention is not limited by these Examples. Incidentally, in Examples, all "%" means "% by mass". Also, the terms "room temperature" in Examples usually mean about 1°C to about 40°C.

### EXAMPLES

### 1. Preparation of Compounds of General formula (I)

### Preparation example 1 2-(3,5-Dimethyl-1H-pyrazol-1-yl)phenol

### a) 1-(2-Methoxyphenyl)-3,5-dimethyl-1H-pyrazole

3.50 g of 2-methoxyphenylhydrazine hydrochloride was dissolved in 60 ml of ethanol and 2.06 ml of acetylacetone was added, followed by heating to reflux for 1 hour. To the reaction mixture was added 150 ml of water, followed by neutralizing with a saturated aqueous sodium carbonate solution and extracting with 150 ml of ethyl acetate. The organic layer was dried over anhydrous magnesium sulfate, subsequently the solvent was distilled off under reduced pressure, and purification by silica gel column chromatography (hexane:ethyl acetate=2:1) afforded 3.88 g of the title compound.
¹H-NMR (CDCl₃); δ (ppm) 2.08 (3H, s), 2.29 (3H, s), 3.78 (3H, s), 5.95 (1H, s), 6.98-7.03 (2H, m), 7.29-7.32 (1H, m), 7.34-7.39 (1H, m).
MS (ESI); m/z 203 (M+H)⁺

### b) 2-(3,5-Dimethyl-1H-pyrazol-1-yl)phenol

3.88 g of 1-(2-methoxyphenyl)-3,5-dimethyl-1H-pyrazole was dissolved in 40 ml of methylene chloride and 32 ml of a 1M solution of boron tribromide in methylene chloride was added, followed by stirring at room temperature for 1.5 hours. The reaction mixture was added to 150 ml of water, followed by neutralizing with 1N-sodium hydroxide and extracting with 150 ml of ethyl acetate. The organic layer was dried over anhydrous magnesium sulfate, subsequently the solvent was distilled off under reduced pressure, and purification by silica gel column chromatography (hexane:ethyl acetate=3:1) afforded 2.83 g of the title compound.
¹H-NMR (CDCl₃); δ (ppm) 2.29 (3H,s), 2.38 (3H, s), 6.02 (1H, s), 6.87-6.6.91 (1H, m), 7.06-7.09 (1H, m), 7.16-7.20 (1H, m).
MS (ESI); m/z 189 (M+H)⁺

### Preparation example 2 2-(3,5-Dimethyl-1H-pyrazol-1-yl)-4-fluorophenol

### a) 2-Amino-4-fluorophenol

300 mg of 4-fluoro-2-nitrophenol was dissolved in 3 ml of ethanol and 120 mg of 10% palladium/carbon was added, followed by stirring under a hydrogen atmosphere at room temperature for 1 hour. The insoluble material was filtered and subsequently the filtrate was distilled off under reduced pressure to afford 211 mg of the title compound.
¹H-NMR (DMSO-d6); δ (ppm) 4.80 (2H, s), 6.09-6.14 (1H, m), 6.34-6.37 (1H, m), 6.53-6.57 (1H, m), 8.93 (1H, s).
MS (FAB); m/z 128 (M+H)⁺

### b) 2-(3,5-Dimethyl-1H-pyrazol-1-yl)-4-fluorophenol

To 100 mg of 2-amino-4-fluorophenol was added 0.8 ml of 5N-hydrochloric acid and a solution of 65 mg of sodium nitrite dissolved in 0.2 ml of water was added dropwise at 0°C, followed by stirring for 30 minutes. Then, a solution of 249 mg of stannous chloride dissolved in 0.46 ml of 5N-hydrochloric acid was added dropwise at 0°C, followed by stirring at 0°C for 30 minutes and then at room temperature for 2 hours. The solvent was distilled off under reduced pressure and 2.5 ml of ethanol and 81 µl of acetylacetone were added, followed by heating to reflux for 4 hours. To the reaction mixture was added 50 ml of water, followed by neutralizing with saturated sodium hydrogen carbonate solution and extracting with 50 ml of ethyl acetate. The organic layer was dried over anhydrous magnesium sulfate, subsequently the solvent was distilled off under reduced pressure, and purification by silica gel column chromatography (hexane:ethyl acetate=2:1) afforded 20.6 mg of the title compound.
¹H-NMR (CDCl₃); δ (ppm) 2.24 (3H, s), 2.33 (3H, s), 5.99 (1H, s), 6.82-6.90 (2H, m), 6.95-6.98 (1H, m).
MS (FAB); m/z 207 (M+H)⁺

### Preparation example 3 2-(1H-Pyrazol-1-yl)phenol

### a) 1-(2-Methoxyphenyl)-1H-pyrazole

200 mg of 2-methoxyphenylhydrazine hydrochloride was dissolved in 5 ml of ethanol and 189 µl of malonaldehyde bisdimethylacetal was added, followed by heating to reflux for 2 hours. To the reaction mixture was added 50 ml of water, followed by neutralizing with a saturated aqueous sodium carbonate solution and extracting with 60 ml of ethyl acetate. The organic layer was dried over anhydrous magnesium sulfate and subsequently the solvent was distilled off under reduced pressure to afford 179.4 mg of the title compound.
¹H-NMR (CDCl₃); δ (ppm) 3.87 (3H,s), 6.42 (1H, d, J=2.4Hz), 7.02-7.07 (2H, m), 7.27-7.32 (1H, m), 7.68-7.72 (2H, m), 8.01 (1H, d, J=2.4Hz).
MS (FAB); m/z 175 (M+H)⁺

### b) 2-(1H-Pyrazol-1-yl)phenol

121 mg of the title compound was afforded from 178 mg of 1-(2-methoxyphenyl)-1H-pyrazole in a manner similar to Preparation example 1b).
¹H-NMR (CDCl₃); δ (ppm) 6.49 (1H, d, J=2.4Hz), 6.88-6.92 (1H, m), 7.08-7.10 (1H, m), 7.14-7.18 (1H, m), 7.35-7.37 (1H, m), 7.72 (1H, s), 7.99 (1H, d, J=2.4Hz).
MS (ESI); m/z 161 (M+H)⁺

### Preparation example 4 2-(5-Hydroxy-3-methyl-1H-pyrazol-1-yl)phenol

### a) 5-Hydroxy-1-(2-methoxyphenyl)-3-methyl-1H-pyrazole

55.1 mg of the title compound was afforded from 150 mg of 2-methoxyphenylhydrazine hydrochloride and 93 µl of methyl acetoacetate in a manner similar to Preparation example 1a).
¹H-NMR (CDCl₃); δ (ppm) 2.11 (3H, s), 3.76 (3H, s), 6.88-6.94 (3H, m), 7.19-7.34 (2H, m).
MS (ESI); m/z 204 (M+H)⁺

### b) 2-(5-Hydroxy-3-methyl-1H-pyrazol-1-yl)phenol

32.4 mg of the title compound was afforded from 52 mg of 5-hydroxy-1-(2-methoxyphenyl)-3-methyl-1H-pyrazole in a manner similar to Preparation example 1b).
¹H-NMR (CDCl₃); δ (ppm) 2.17 (3H, s), 5.30 (1H, s), 6.88-6.95 (2H, m), 7.15 (1H, t, J=7.6Hz), 7.37 (1H, d, J=7.6Hz).
MS (FAB); m/z 191 (M+H)⁺

### Preparation example 5

### 2-(5-Methyl-3-trifluoromethyl-1H-pyrazol-1-yl)phenol

### a) 1-(2-Methoxyphenyl)-5-methyl-3-trifluoromethyl-1H-pyrazole

300 mg of 2-methoxyphenylhydrazine hydrochloride was dissolved in 1.3 ml of 2-methoxyethanol, and 2.5 ml of acetic acid and 208 µl of 1,1,1-trifluoro-2,4-pentanedione were added, followed by heating to reflux for 1 hour and 40 minutes. The solvent was distilled off under reduced pressure, 50 ml of ethyl acetate was added, the organic layer washed with 50 ml of saturated sodium hydrogen carbonate solution and 50 ml of saturated brine was dried over anhydrous magnesium sulfate, and the solvent was distilled off under reduced pressure to afford 485.6 mg of the title compound.
¹H-NMR (CDCl₃); δ (ppm) 2.14 (3H, s), 3.78 (3H, s), 6.40 (1H, s), 7.00-7.07 (2H, m), 7.31-7.33 (1H, m), 7.41-7.45 (1H, m).
MS (ESI); m/z 257 (M+H)⁺

### b) 2-(5-Methyl-3-(trifluoromethyl)-1H-pyrazol-1-yl)phenol

320.1 mg of the title compound was afforded from 1-(2-methoxyphenyl)-5-methyl-3-(trifluoromethyl)-1H-pyrazole in a manner similar to Preparation example 1b).
¹H-NMR (CDCl₃); δ (ppm) 2.41 (3H, s), 6.52 (1H, s), 6.95-6.99 (1H, m), 7.11-7.13 (1H, m), 7.20-7.24 (1H, m), 7.28-7.32 (1H, m).
MS (FAB); m/z 243 (M+H)⁺

### Preparation example 6 2-(3,5-Bistrifluoromethyl-1H-pyrazol-1-yl)phenol

### a) 1-(2-Methoxyphenyl)-3,5-bistrifluoromethyl-1H-pyrazole

The title compound was afforded from 300 mg of 2-methoxyphenylhydrazine hydrochloride and 243 µl of hexafluoroacetylacetone in a manner similar to Preparation example 5a).
¹H-NMR (CDCl₃); δ (ppm) 3.77 (3H, s), 7.01-7.06 (3H, m), 7.33 (1H, d, J=7.6Hz), 7.49 (1H, d, J=7.6Hz).
MS (ESI); m/z 311 (M+H)⁺

### b) 2-(3,5-Bistrifluoromethyl-1H-pyrazol-1-yl)phenol

455.6 mg of the title compound was afforded from 1-(2-methoxyphenyl)-3,5-bistrifluoromethyl-1H-pyrazole obtained in the above a) in a manner similar to Preparation example 1b).
¹H-NMR (CDCl₃); δ (ppm) 6.99-7.04 (1H, m), 7.07-7.08 (1H, m), 7.10 (1H, s), 7.32-7.41 (2H, m).
MS (FAB); m/z 297 (M+H)⁺

### Preparation example 7 2-(3-Methyl-1H-pyrazol-1-yl)phenol

### a) 1-(2-Methoxyphenyl)-3-methyl-1H-pyrazole

115.1 mg of the title compound was afforded from 200 mg of 2-methoxyphenylhydrazine hydrochloride and 151 µl of 4,4-dimethoxybutan-2-one in a manner similar to Preparation example 1 a).
¹H-NMR (CDCl₃); δ (ppm) 2.31 (3H, s), 3.80 (3H, s), 6.14 (1H, d, J=2.4Hz), 6.95-6.99 (2H, m), 7.18-7.22 (1H, m), 7.61-7.63 (1H, m), 7.84 (1H, d, J=2.4Hz).
MS (FAB); m/z 189 (M+H)⁺

### b) 2-(3-Methyl-1H-pyrazol-1-yl)phenol

76 mg of the title compound was afforded from 115 mg of 1-(2-methoxyphenyl)-3-methyl-1H-pyrazole in a manner similar to Preparation example 1b).
¹H-NMR (CDCl₃); δ (ppm) 2.36 (3H, s), 6.24 (1H, d, J=2.4Hz), 6.85-6.89 (1H, m), 7.05-7.14 (2H, m), 7.29-7.31 (1H, m), 7.86 (1H, d, J=2.4Hz).
MS (FAB); m/z 175 (M+H)⁺

### Preparation example 8 2-(5-Methyl-1H-pyrazol-1-yl)phenol

### a) 1-(2-Methoxyphenyl)-5-methyl-1H-pyrazole

70 mg of the title compound was afforded from 200 mg of 2-methoxyphenylhydrazine hydrochloride and 151 µl of 4,4-dimethoxybutan-2-one in a manner similar to Preparation example 1a).
¹H-NMR (CDCl₃); δ (ppm) 2.07 (3H, s), 3.71 (3H, s), 6.09 (1H, s), 6.94-6.99 (2H, m), 7.24-7.26 (1H, m), 7.31-7.35 (1H, m), 7.52 (1H, s).
MS (FAB); m/z 189 (M+H)⁺

### b) 2-(5-Methyl-1H-pyrazol-1-yl)phenol

45.5 mg of the title compound was afforded from 69 mg of 1-(2-methoxyphenyl)-5-methyl-1H-pyrazole in a manner similar to Preparation example 1b).
¹H-NMR (CDCl₃); δ (ppm) 2.41 (3H, s), 6.27 (1H, d, J=1.6Hz), 6.90-6.94 (1H, m), 7.10-7.12 (1H, m), 7.19-7.24 (2H, m), 7.66 (1H, d, J=1.6Hz).
MS (FAB); m/z 175 (M+H)⁺

### Preparation example 9 2-(3,4,5-Trimethyl-1H-pyrazol-1-yl)phenol

### a) 1-(2-Methoxyphenyl)-3,4,5-trimethyl-1H-pyrazole

200 mg of 2-methoxyphenylhydrazine hydrochloride was dissolved in 4 ml of ethanol and 134 µl of 3-methyl-2,4-pentanedione was added, followed by heating to reflux for 3 hours. To the reaction mixture was added 50 ml of water, followed by neutralizing with a saturated aqueous sodium carbonate solution and extracting with 60 ml of ethyl acetate. The organic layer was dried over anhydrous magnesium sulfate and subsequently the solvent was distilled off under reduced pressure to afford 209.8 mg of the title compound.
¹H-NMR (CDCl₃); δ (ppm) 1.98 (3H, s), 2.02 (3H, s), 2.24 (3H, s), 3.80 (3H, s), 6.99-7.04 (2H, m), 7.29-7.31 (1H, m), 7.34-7.39 (1H, m).
MS (FAB); m/z 217 (M+H)⁺

### b) 2-(3,4,5-Trimethyl-1H-pyrazol-1-yl)phenol

104 mg of the title compound was afforded from 209 mg of 1-(2-methoxyphenyl)-3,4,5-trimethyl-1H-pyrazole in a manner similar to Preparation example 1b).
¹H-NMR (CDCl₃); δ (ppm) 1.99 (3H, s), 2.25 (3H, s), 2.31 (3H, s), 6.90 (1H, t, J=8.0Hz), 7.08 (1H, d, J=8.0Hz), 7.06-7.20 (2H, m), 9.89 (1H, s).
MS (ESI); m/z 203 (M+H)⁺

### Preparation example 10 2-(5-Amino-3-tert-butyl-1H-pyrazol-1-yl)phenol

### a) 3-tert-Butyl-1-(2-methoxyphenyl)-1H-pyrazol-5-amine

310.3 mg of the title compound was afforded from 300 mg of 2-methoxyphenylhydrazine hydrochloride and 215 mg of 4,4-dimethyl-3-oxopentanenitrile and 40 µl of acetic acid in a manner similar to Preparation example 1a).
¹H-NMR (CDCl₃); δ (ppm) 1.32(9H, s), 3.80 (2H, s), 3.86 (3H, s), 5.51 (1H, s), 7.01-7.08 (2H, m), 7.30-7.35 (1H, m), 7.45-7.47 (1H, m).
MS (FAB); m/z 246 (M+H)⁺

### b) 2-(5-Amino-3-tert-butyl-1H-pyrazol-1-yl)phenol

66.2 mg of the title compound was afforded from 100 mg of 3-tert-butyl-1-(2-methoxyphenyl)-1H-pyrazol-5-amine in a manner similar to Preparation example 1b).
¹H-NMR (CDCl₃); δ (ppm) 1.30 (9H, s), 3.93 (2H, s), 5.55 (1H, s), 6.90 (1H, dt, J=1.6, 8.0Hz), 7.08 (1H, dd, J=1.6, 8.0Hz), 7.17 (1H, dt, J=1.6, 8.0Hz), 7.47 (1H, dd, J=1.6, 8.0Hz), 10.39 (1H, brs).
MS (FAB); m/z 232 (M+H)⁺

### Preparation example 11 4-Chloro-2-(3,5-dimethyl-1H-pyrazol-1-yl)phenol

### a) 1-(5-Chloro-2-methoxyphenyl)-3,5-dimethyl-1H-pyrazole

104.8 mg of the title compound was afforded from 388 mg of 5-chloro-2-methoxyaniline hydrochloride in a manner similar to Preparation example 2b).
¹H-NMR (CDCl₃); δ (ppm) 2.11 (3H, s), 2.29 (3H, s), 3.79 (3H, s), 5.96 (1H, s), 6.93 (1H, dd, J=2.4, 7.2Hz), 7.33-7.35 (2H, m).
MS (FAB); m/z 237 (M+H)⁺

### b) 4-Chloro-2-(3,5-dimethyl-1H-pyrazol-1-yl)phenol

67.3 mg of the title compound was afforded from 104.8 mg of 1-(5-chloro-2-methoxyphenyl)-3,5-dimethyl-1H-pyrazole in a manner similar to Preparation example 1b).
¹H-NMR (CDCl₃); δ (ppm) 2.30 (3H, s), 2.43 (3H, s), 6.05 (1H, s), 7.02 (1H, d, J=8.8Hz), 7.14-7.20 (2H, m), 10.08 (1H, s).
MS (FAB); m/z 223 (M+H)⁺

### Preparation example 12 2-Chloro-6-(3,5-dimethyl-1H-pyrazol-1-yl)phenol

### a) 1-(3-Chloro-2-methoxyphenyl)-3,5-dimethyl-1H-pyrazole

30.6 mg of the title compound was afforded from 158 mg of 3-chloro-o-anisidine in a manner similar to Preparation example 2b).
¹H-NMR (CDCl₃); δ (ppm) 2.14 (3H, s), 2.29 (3H, s), 3.49 (3H, s), 5.99 (1H,s), 7.12 (1H, t, J=8.0Hz), 7.27-7.31 (1H, m), 7.43-7.46 (1H, m).
MS (ESI); m/z 236 (M+H)⁺

### b) 2-Chloro-6-(3,5-dimethyl-1H-pyrazol-1-yl)phenol

27.4 mg of the title compound was afforded from 63.4 mg of 1-(3-chloro-2-methoxyphenyl)-3,5-dimethyl-1H-pyrazole in a manner similar to Preparation example 1b).
¹H-NMR (CDCl₃); δ (ppm) 2.30 (3H, s), 2.40 (3H, s), 6.05 (1H, s), 6.86 (1H, t, J=8.0Hz),7.13-7.15 (1H, m), 7.26-7.31 (1H, m), 10.66 (1H, s).
MS (FAB); m/z 223 (M+H)⁺

### Preparation example 13 2-(4-Chloro-3,5-dimethyl-1H-pyrazol-1-yl)phenol

### a) 4-Chloro-1-(2-methoxyphenyl)-3,5-dimethyl-1H-pyrazole

193.9 mg of the title compound was afforded from 174.6 mg of 2-methoxyphenylhydrazine hydrochloride and 114 µl of 3-chloropentane-2, 4-dione in a manner similar to Preparation example 1a).
¹H-NMR (CDCl₃); δ (ppm) 2.09 (3H,s), 2.29 (3H, s), 3.81 (3H, s), 7.00-7.06 (2H, m), 7.30 (1H, dd, J=1.6, 7.6Hz), 7.38-7.43 (1H, m).
MS (FAB); m/z 237 (M+H)⁺

### b) 2-(4-Chloro-3,5-dimethyl-1H-pyrazol-1-yl)phenol

151.9 mg of the title compound was afforded from 193 mg of 4-chloro-1-(2-methoxyphenyl)-3,5-dimethyl-1H-pyrazole in a manner similar to Preparation example 1b).
¹H-NMR (CDCl₃); δ (ppm) 2.30 (3H, s), 2.37 (3H, s), 6.91-6.95 (1H, m), 7.09 (1H, dd, J=1.6, 8.0Hz), 7.17 (1H, dd, J=1.6, 8.0Hz), 7.21-7.25 (1H, m), 9.23 (1H, s).
MS (FAB); m/z 223 (M+H)⁺

### Preparation example 14 2-(3,5-Diethyl-1H-pyrazol-1-yl)phenol

### a) 3,5-Diethyl-1-(2-methoxyphenyl)-1H-pyrazole

209.8 mg of the title compound was afforded from 174.6 mg of 2-methoxyphenylhydrazine hydrochloride and 135.5 µl of 3,5-heptanedione in a manner similar to Preparation example 1a).
¹H-NMR (CDCl₃); δ (ppm) 1.14-1.18 (3H, m), 1.25-1.31 (3H, m), 2.42 (2H, q, J=7.2Hz), 2.67-2.73 (2H, m), 3.78 (3H, s), 6.03 (1H, s), 6.99-7.04 (2H, m), 7.31-7.40 (2H, m).
MS (FAB); m/z 231 (M+H)⁺

### b) 2-(3,5-Diethyl-1H-pyrazol-1-yl)phenol

159.2 mg of the title compound was afforded from 207 mg of 3,5-diethyl-1-(2-methoxyphenyl)-1H-pyrazole in a manner similar to Preparation example 1b).
¹H-NMR (CDCl₃); δ (ppm) 1.24-1.31(6H, m), 2.69 (2H, q, J=7.6Hz), 2.76 (2H, q, J=7.6Hz), 6.11 (1H, s), 6.90 (1H, q, J=7.6Hz), 7.09 (1H, d, J=7.6Hz), 7.19-7.22 (2H, m), 9.69 (1H, s).
MS (FAB); m/z 217 (M+H)⁺

### Preparation example 15 3-(3,5-Dimethyl-1H-pyrazol-1-yl)benzene-1,2-diol

### a) 1-(2,3-Dimethoxyphenyl)-3,5-dimethyl-1H-pyrazole

189 mg of the title compound was afforded from 306 mg of 2,3-dimethoxyaniline in a manner similar to Preparation example 2b).
¹H-NMR (CDCl₃); δ (ppm) 2.14 (3H, s), 2.29 (3H, s), 3.53 (3H, s), 3.91 (3H, s), 5.96 (1H, s), 6.96-6.70 (2H, m), 7.12 (1H, t, J=8.0Hz).
MS (FAB); m/z 233 (M+H)⁺

### b) 3-(3,5-Dimethyl-1H-pyrazol-1-yl)benzene-1,2-diol

28.9 mg of the title compound was afforded from 186 mg of 1-(2,3-dimethoxyphenyl)-3,5-dimethyl-1H-pyrazole in a manner similar to Preparation example 1b).
¹H-NMR (CDCl₃); δ (ppm) 2.26 (3H, s), 2.38 (3H, s), 5.99 (1H, s), 6.75-6.85 (3H, m).
MS (FAB); m/z 205 (M+H)⁺

### Preparation example 16 2-(3,5-Dimethyl-1H-pyrazol-1-yl)benzene-1,4-diol

### a) 1-(2,5-Dimethoxyphenyl)-3,5-dimethyl-1H-pyrazole

260.3 mg of the title compound was afforded from 306 mg of 2,5-dimethoxyaniline in a manner similar to Preparation example 2b).
¹H-NMR (CDCl₃); δ (ppm) 2.11 (3H, s), 2.30 (3H, s), 3.73 (3H, s), 3.78 (3H, s), 5.96 (1H, s), 6.91-6.93 (3H, m).

### b) 2-(3,5-Dimethyl-1H-pyrazol-1-yl)benzene-1,4-diol

162.6 mg of the title compound was afforded from 260.3 mg of 1-(2,5-dimethoxyphenyl)-3,5-dimethyl-1H-pyrazole in a manner similar to Preparation example 1b).
¹H-NMR (DMSO-d6); δ (ppm) 2.07 (3H, s), 2.14 (3H, s), 5.93 (1H, s), 6.56 (1H, d, J=2.8Hz), 6.66-6.69 (1H, m), 6.80 (1H, d, J=8.8Hz), 9.02 (1H, s), 9.17 (1H, s).
MS (FAB); m/z 205 (M+H)⁺

### Preparation example 17 2-(4-Ethyl-3,5-dimethyl-1H-pyrazol-1-yl)phenol

### a) 4-Ethyl-1-(2-methoxyphenyl)-3,5-dimethyl-1H-pyrazole

236.6 mg of the title compound was afforded from 200 mg of 2-methoxyphenylhydrazine hydrochloride and 155 µl of 3-ethyl-2,4-pentanedione in a manner similar to Preparation example 1a).
¹H-NMR (CDCl₃); δ (ppm) 1.13 (3H, t, J=7.6Hz), 2.03 (3H, s), 2.27 (3H, s), 2.42 (2H, q, J=7.6Hz), 3.79 (3H, s), 6.99-7.04 (2H, m), 7.30-7.39 (2H, m).
MS (FAB); m/z 231 (M+H)⁺

### b) 2-(4-Ethyl-3,5-dimethyl-1H-pyrazol-1-yl)phenol

196.2 mg of the title compound was afforded from 232 mg of 4-ethyl-1-(2-methoxyphenyl)-3,5-dimethyl-1H-pyrazole in a manner similar to Preparation example 1b).
¹H-NMR (CDCl₃); δ (ppm) 1.12 (3H, t, J=7.6Hz), 2.27 (3H, s), 2.33 (3H, s), 2.43 (2H, q, J=7.6Hz), 6.88-6.92 (1H, m), 7.08-7.10 (1H, m), 7.06-7.19 (2H, m), 9.90 (1H, s).
MS (FAB); m/z 217 (M+H)⁺

### Preparation example 18 5-Fluoro-2-(3,4,5-trimethyl-1H-pyrazol-1-yl)phenol

114.7 mg of the title compound was afforded from 201.2 mg of 2-amino-5-fluorophenol and 184 µl of 3-methyl-2,4-pentanedione in a manner similar to Preparation example 1b).
¹H-NMR (CDCl₃); δ (ppm) 1.99 (3H, s), 2.23 (3H, s), 2.28 (3H, s), 6.59-6.64 (1H, m), 6.78-6.81 (1H, m), 7.10-7.14 (1H, m).
MS (ESI); m/z 221 (M+H)⁺

### Preparation example 19

### 2-(4-Chloro-3,5-dimethyl-1H-pyrazol-1-yl)benzene-1,4-diol

### a) 4-Chloro-1-(2,5-dimethoxyphenyl)-3,5-dimethyl-1H-pyrazole

333.7 mg of the title compound was afforded from 306 mg of 2,5-dimethoxyaniline and 228 µl of 3-chloropentane-2,4-dione in a manner similar to Preparation example 2b).
¹H-NMR (CDCl₃); δ (ppm) 2.10 (3H, s), 2.29 (3H, s), 3.74 (3H, s), 3.78 (3H, s), 6.94-6.97 (2H, m), 7.26 (1H, s).
MS (FAB); m/z 267 (M+H)⁺

### b) 2-(4-Chloro-3,5-dimethyl-1H-pyrazol-1-yl)benzene-1,4-diol

184.8 mg of the title compound was afforded from 329 mg of 4-chloro-1-(2,5-dimethoxyphenyl)-3,5-dimethyl-1H-pyrazole in a manner similar to Preparation example 1b).
¹H-NMR (DMSO-d6); δ (ppm) 2.05 (3H, s), 2.15 (3H, s), 6.58 (1H, d, J=2.8Hz), 6.72 (1H, dd, J=2.8, 8.8Hz), 6.82 (1H, d, J=8.8Hz), 9.11 (1H, s), 9.33 (1H, s).
MS (FAB); m/z 239 (M+H)⁺

Preparation example 20 4-Fluoro-2-(3,4,5-trimethyl-1H-pyrazol-1-yl)phenol

84.8 mg of the title compound was afforded from 111 mg of 2-amino-4-fluorophenol and 102 µl of 3-methyl-2,4-pentanedione in a manner similar to Preparation example 2b).
¹H-NMR (CDCl₃); δ (ppm) 1.99 (3H, s), 2.23 (3H, s), 2.24 (3H, s), 2.34 (3H, s), 6.87-6.95 (2H, m), 7.00-7.03 (1H, m), 9.90 (1H, s).
MS (FAB); m/z 221 (M+H)⁺

### Preparation example 21

### 2-(4-Chloro-3,5-dimethyl-1H-pyrazol-1-yl)-5-fluorophenol

62 mg of the title compound was afforded from 100 mg of 2-amino-5-fluorophenol and 90 µl of 3-chloropentane-2,4-dione in a manner similar to Preparation example 2b).
¹H-NMR (CDCl₃); δ (ppm) 2.29 (3H, s), 2.35 (3H, s), 6.62-6.67 (1H, m), 6.78-6.81 (1H, m), 7.10-7.14 (1H, m), 9.44 (1H, s).
MS (FAB); m/z 241 (M+H)⁺

### Preparation example 22 Ethyl

### 1-(2-hydroxyphenyl)-3,5-dimethyl-1H-pyrazol-4-carboxylate

### a) Ethyl 1-(2-methoxyphenyl)-3,5-dimethyl-1H-pyrazol-4-carboxylate

82.6 mg of the title compound was afforded from 175 mg of 2-methoxyphenylhydrazine hydrochloride and 156 µl of ethyl 2-acetyl-3-oxobutanoate in a manner similar to Preparation example 1a).
¹H-NMR (CDCl₃); δ (ppm) 1.38 (3H, t, J=7.6Hz), 2.33 (3H, s), 2.50 (3H, s), 80 (3H, s), 4.32 (2H, q, J=7.6Hz), 7.02-7.08 (2H, m), 7.30-7.32 (1H, m), 7.41-7.45 (1H, m).
MS (ESI); m/z 275 (M+H)⁺

### b) Ethyl 1-(2-hydroxyphenyl)-3,5-dimethyl-1H-pyrazol-4-carboxylate

21 mg of the title compound was afforded from 82 mg of ethyl 1-(2-methoxyphenyl)-3,5-dimethyl-1H-pyrazol-4-carboxylate in a manner similar to Preparation example 1b).
¹H-NMR (CDCl₃); δ (ppm) 1.39 (3H, t, J=7.2Hz), 2.50 (3H, s), 2.61 (3H, s), 4.34 (2H, q, J=7.2Hz), 6.96 (1H, t, J=8.4Hz), 7.10 (1H, d, J=8.4Hz), 7.17 (1H, d, J=8.4Hz), 7.26-7.30 (1H, m), 8.76 (1H, s).
MS (FAB); m/z 261 (M+H)⁺

### Preparation example 23 Methyl

### 3-(1-(2-hydroxyphenyl)-3,5-dimethyl-1H-pyrazol-4-yl)propanoate

### a) Methyl 3-(1-(2-methoxyphenyl)-3,5-dimethyl-1H-pyrazol-4-yl)propanoate

96 mg of the title compound was afforded from 174.6 mg of 2-methoxyphenylhydrazine hydrochloride and 175 µl of methyl 4-acetyl-5-oxohexanoate in a manner similar to Preparation example 1a).
¹H-NMR (CDCl₃); δ (ppm) 2.04 (3H, s), 2.27 (3H, s), 2.49-2.54 (2H, m), 2.74-2.78 (2H, m), 3.68 (3H, s), 3.79 (3H, s), 6.99-7.02 (2H, m), 7.28-7.31 (1H, m), 7.35-7.39 (1H, m).
MS (FAB); m/z 289 (M+H)⁺

### b) Methyl 3-(1-(2-hydroxyphenyl)-3,5-dimethyl-1H-pyrazol-4-yl)propanoate

36.8 mg of the title compound was afforded from 96 mg of methyl 3-(1-(2-methoxyphenyl)-3,5-dimethyl-1H-pyrazol-4-yl)propanoate in a manner similar to Preparation example 1b).
¹H-NMR (CDCl₃); δ (ppm) 2.28 (3H, s), 2.34 (3H, s), 2.51 (2H, t, J=8.0Hz), 2.77 (2H, t, J=8.0Hz), 3.69 (3H, s), 6.91 (1H, t, J=6.8Hz), 7.08-7.10 (1H, m), 7.17-7.26 (2H, m), 9.73 (1H, s).
MS (ESI); m/z 275 (M+H)⁺

### Preparation example 24 2-(4-Butyl-3,5-dimethyl-1H-pyrazol-1-yl)phenol

### a) 4-Butyl-1-(2-methoxyphenyl)-3,5-dimethyl-1H-pyrazole

273 mg of the title compound was afforded from 175 mg of 2-methoxyphenylhydrazine hydrochloride and 168 µl of 3-n-butyl 2,4-pentanedione in a manner similar to Preparation example 1a).
¹H-NMR (CDCl₃); δ (ppm) 0.94 (3H, t, J=7.2Hz), 1.32-1.39 (2H, m), 1.45-1.50 (2H, m), 2.01 (3H, m), 2.26 (3H, s), 2.39 (2H, q, J=7.2Hz), 3.79 (3H, s), 6.99-7.04 (2H, m), 7.30-7.38 (2H, m).
MS (FAB); m/z 259 (M+H)⁺

### b) 2-(4-Butyl-3,5-dimethyl-1H-pyrazol-1-yl)phenol

208.8 mg of the title compound was afforded from 273 mg of 4-butyl-1-(2-methoxyphenyl)-3,5-dimethyl-1H-pyrazole in a manner similar to Preparation example 1b).
¹H-NMR (CDCl₃); δ (ppm) 0.95 (3H, t, J=7.6Hz), 1.33-1.39 (2H, m), 1.43-1.48 (2H, m), 2.26 (3H, s), 2.32 (3H, s), 2.40 (2H, t, J=7.6Hz), 6.88-6.92 (1H, m), 7.08 (1H, d, J=8.4Hz), 7.16-7.20 (2H, m), 9.93 (1H, s).
MS (FAB); m/z 245 (M+H)⁺

### Preparation example 25 2-(3,5-Dimethyl-1H-pyrazol-1-yl)-5-fluorophenol

### a) 2-Amino-5-fluorophenol

252.5 mg of the title compound was afforded from 314 mg of 5-fluoro-2-nitrophenol in a manner similar to Preparation example 2a).
¹H-NMR (DMSO-d6); δ (ppm) 6.33-6.38 (1H, m), 6.45-6.48 (1H, m), 6.51-6.55 (1H, m).
MS (FAB); m/z 128 (M+H)⁺

### b) 2-(3,5-Dimethyl-1H-pyrazol-1-yl)-5-fluorophenol

89.6 mg of the title compound was afforded from 150 mg of 2-amino-5-fluorophenol in a manner similar to Preparation example 2b).
¹H-NMR (CDCl₃); δ (ppm) 2.29 (3H, s), 2.37 (3H, s), 6.03 (1H, s), 6.60-6.65 (1H, m), 6.78-6.81 (1H, m), 7.13-7.16 (1H, m).
MS (ESI); m/z 207 (M+H)⁺

### Preparation example 26 5-Chloro-2-(3,5-dimethyl-1H-pyrazol-1-yl)phenol

168.8 mg of the title compound was afforded from 287 mg of 2-amino-5-chlorophenol in a manner similar to Preparation example 2b).
¹H-NMR (CDCl₃); δ (ppm) 2.30 (3H, s), 2.40 (3H, s), 6.05 (1H, s), 6.87-6.90 (1H, m), 7.10-7.14 (2H, m), 10.23 (1H, s).
MS (FAB); m/z 223 (M+H)⁺

### Preparation example 27 2-(3,5-Dimethyl-1H-pyrazol-1-yl)-3-nitrophenol

75.4 mg of the title compound was afforded from 308 mg of 2-amino-3-nitrophenol in a manner similar to Preparation example 2b).
¹H-NMR (CD₃OD); δ (ppm) 2.14 (3H, s), 2.19 (3H, s), 6.04 (1H, s), 7.27 (1H, dd, J=1.6, 7.6Hz), 7.44 (1H, dd, J=1.6, 7.6Hz), 7.50 (1H, t, J=7.6Hz).
MS (FAB); m/z 234 (M+H)⁺

### Preparation example 28 2-(3,5-Dimethyl-1H-pyrazol-1-yl)-5-nitrophenol

101 mg of the title compound was afforded from 308 mg of 2-amino-5-nitrophenol in a manner similar to Preparation example 2b).
¹H-NMR (CDCl₃); δ (ppm) 2.33 (3H, s), 2.50 (3H, s), 6.13 (1H, s), 7.38 (1H, d, J=7.6Hz), 7.79-7.81 (1H, m), 7.95 (1H, s).
M S(ESI); m/z 234 (M+H)⁺

### Preparation example 29

### 3-(1-(2-Hydroxyphenyl)-3,5-dimethyl-1H-pyrazol-4-yl)propionic acid

29.9 mg of methyl 3-(1-(2-hydroxyphenyl)-3,5-dimethyl-1Hpyrazol-4-yl)propanoate was dissolved in 0.6 ml of methanol and 0.29 ml of 1N-sodium hydroxide was added, followed by stirring at room temperature for 3.5 hours. To the reaction mixture was added 20 ml of water, followed by neutralizing with 1N-hydrochloric acid and extracting with 20 ml of ethyl acetate. The organic layer was dried over anhydrous magnesium sulfate and subsequently the solvent was distilled off under reduced pressure to afford 7.5 mg of the title compound.
¹H-NMR (CDCl₃); δ (ppm) 2.29 (3H, s), 2.34 (3H, s), 2.56 (2H, t, J=7.6Hz), 2.78 (2H, t, J=7.6Hz), 6.89-6.93 (1H, m), 7.08-7.10 (1H, m), 7.16-7.22 (2H, m).
MS (FAB); m/z 261 (M+H)⁺

### Preparation example 30 5-Chloro-2-(3,4,5-trimethyl-1H-pyrazol-1-yl)phenol

152 mg of the title compound was afforded from 287 mg of 2-amino-5-chlorophenol and 233 µl of 3-methyl-2,4-pentanedione in a manner similar to Preparation example 2b).
¹H-NMR (CDCl₃); δ (ppm) 1.99 (3H, s), 2.24 (3H, s), 2.31 (3H, s), 6.86-6.89 (1H, m), 7.09-7.11 (2H, m).
MS (FAB); m/z 227 (M+H)⁺

### Preparation example 31 5-Amino-2-(3,5-dimethyl-1H-pyrazol-1-yl)phenol

86 mg of 2-(3,5-dimethyl-1H-pyrazol-1-yl)-5-nitrophenol was dissolved in 1.7 ml of ethanol and 43 mg of 10% palladium/carbon was added, followed by stirring under a hydrogen atmosphere at room temperature for 45 minutes. The insoluble material was filtered and subsequently the solvent was distilled off under reduced pressure to afford 30.4 mg of the title compound.
¹H-NMR (CDCl₃); δ (ppm) 2.28 (3H, s), 2.33 (3H, s), 3.72 (2H, s), 5.99 (1H, s), 6.22 (1H, dd, J=2.4, 8.4Hz), 6.40 (1H, d, J=2.4Hz), 6.97 (1H, d, J=8.4Hz), 9.27 (1H, s).
MS (FAB); m/z 204 (M+H)⁺

### Preparation example 32 5-Nitro-2-(3,4,5-trimethyl-1H-pyrazol-1-yl)phenol

289.5 mg of the title compound was afforded from 308 mg of 2-amino-5-nitrophenol and 233 µl of 3-methyl-2,4-pentanedione in a manner similar to Preparation example 2b).
¹H-NMR (CDCl₃); δ (ppm) 2.02 (3H, s), 2.24 (3H, s), 2.40 (3H, s), 7.33 (1H, d, J=8.8Hz), 7.79 (1H, dd, J=2.4, 8.8Hz), 7.94 (1H, d, J=2.4Hz).
MS (FAB); m/z 248 (M+H)⁺

### Preparation example 33 4-(3,5-Dimethyl-1H-pyrazol-1-yl)benzene-1,3-diol

### a) 2,4-Dimethoxyphenylboronic acid

576 µl of 1-bromo-2,4-dimethoxybenzene was dissolved in 5.8 ml of tetrahydrofuran and 3 ml of a 1.6 mol/l solution of n-butyllithium in hexane was added dropwise under an argon atmosphere at -78°C. Then, 1.1 ml of triisopropyl borate was added, followed by stirring at -78°C for 40 minutes and then stirring at room temperature for 2 hours. To the reaction mixture were added 40 ml of water and 1 ml of 5N-hydrochloric acid, followed by extracting with 50 ml of ethyl acetate. The organic layer was dried over anhydrous magnesium sulfate, subsequently the solvent was distilled off under reduced pressure, and purification using silica gel column chromatography (hexane:ethyl acetate=2:1) afforded 610.2 mg of the title compound.
¹H-NMR (CDCl₃); δ (ppm) 3.85 (3H, s), 3.89 (3H, s), 5.81 (2H, s), 6.46 (1H, s), 6.56 (1H, dd, J=2.0, 8.4Hz), 7.77 (1H, d, J=8.4Hz).

### b) 1-(2,4-Dimethoxyphenyl)-3,5-dimethyl-1H-pyrazole

610.2 mg of 2,4-dimethoxyphenylboronic acid was dissolved in 6 ml of methylene chloride, 387 mg of 3,5-dimethylpyrazole, 730 mg of copper(II) acetate and 948 µl of pyridine were added, followed by stirring at room temperature overnight. To the reaction mixture was added 60 ml of water, followed by extracting with 60 ml of ethyl acetate. The organic layer was dried over anhydrous magnesium sulfate, subsequently the solvent was distilled off under reduced pressure, and purification using silica gel column chromatography (hexane:ethyl acetate=3:2) afforded 81.7 mg of the title compound.
¹H-NMR (CDCl₃); δ (ppm) 2.07 (3H, s), 2.29 (3H, s), 3.77 (3H, s), 3.85 (3H, s), 5.94 (1H, s), 6.52-6.54 (2H, m), 7.22-7.24 (1H, m).
MS (FAB); m/z 233 (M+H)⁺

### c) 4-(3,5-Dimethyl-1H-pyrazol-1-yl)benzene-1,3-diol

118.4 mg of 1-(2,4-dimethoxyphenyl)-3,5-dimethyl-1H-pyrazole was dissolved in 2.3 ml of methylene chloride and 1.7 ml of a 1M solution of boron tribromide in methylene chloride was added, followed by stirring at room temperature for 1 hour. The reaction mixture was added to 30 ml of water, followed by neutralizing with 1N-sodium hydroxide and extracting with 50 ml of ethyl acetate. The organic layer was dried over anhydrous magnesium sulfate, subsequently the solvent was distilled off under reduced pressure, and purification using preparative thin-layer silica gel column chromatography (hexane:ethyl acetate=1:2) afforded 71.7 mg of the title compound.
¹H-NMR (CDCl₃); δ (ppm) 2.29 (3H, s), 2.33 (3H,s), 6.01 (1H, s), 6.37 (1H, dd, J=2.8, 8.8Hz), 6.53 (1H, d, J=2.8Hz), 7.03 (1H, d, J=8.8Hz).
MS (ESI); m/z 205 (M+H)⁺

### Preparation example 34 5-Amino-2-(3,4,5-trimethyl-1H-pyrazol-1-yl)phenol

118.4 mg of the title compound was afforded from 200 mg of 5-nitro-2-(3,4,5-trimethyl-1H-pyrazol-1-yl)phenol in a manner similar to Preparation example 31.
¹H-NMR (CDCl₃); δ (ppm) 1.97 (3H, s), 2.23 (3H, s), 2.24 (3H, s), 3.70 (2H, s), 6.22 (1H, dd, J=2.4, 8.4Hz), 6.40 (1H, d, J=2.4Hz), 6.95 (1H, d, J=8.4Hz), 9.34 (1H, s).
MS (FAB); m/z 218 (M+H)⁺

### Preparation example 35 Methyl

### 4-(3,5-dimethyl-1H-pyrazol-1-yl)-3-hydroxybenzenecarboxylate

239.4 mg of the title compound was afforded from 334 mg of methyl 4-amino-3-hydroxybenzenecarboxylate in a manner similar to Preparation example 2b).
¹H-NMR (CDCl₃); δ (ppm) 2.31 (3H, s), 2.45 (3H, s), 3.92 (3H, s), 6.08 (1H, s), 7.26-7.29 (1H, m), 7.60 (1H, dd, J=1.6, 8.0Hz), 7.76 (1H, d, J=1.6Hz).
MS (ESI); m/z 247 (M+H)⁺

### Preparation example 36 3-Amino-2-(3,5-dimethyl-1H-pyrazol-1-yl)phenol

38.9 mg of the title compound was afforded from 56.6 mg of 2-(3,5-dimethyl-1H-pyrazol-1-yl)-3-nitrophenol in a manner similar to Preparation example 31.
¹H-NMR (CDCl₃); δ (ppm) 2.14 (3H, s), 2.25 (3H, s), 3.57 (2H, s), 6.02 (1H, s), 6.32-6.37 (2H, m), 7.02 (1H, t, J=8.0Hz).
MS (FAB); m/z 204 (M+H)⁺

### Preparation example 37

### 4-(3,5-Dimethyl-1H-pyrazol-1-yl)-3-hydroxybenzene carboxylic acid

100 mg of methyl 4-(3,5-dimethyl-1H-pyrazol-1-yl)-3-hydroxybenzenecarboxylate was dissolved in 1 ml of methanol and 1.6 ml of 1N-sodium hydroxide was added, followed by stirring at room temperature for 3.5 hours. To the reaction mixture was added 20 ml of water, followed by neutralizing with 1N-hydrochloric acid and extracting with 20 ml of ethyl acetate. The organic layer was dried over anhydrous magnesium sulfate and subsequently the solvent was distilled off under reduced pressure to afford 40.8 mg of the title compound.
¹H-NMR (CD₃OD); δ (ppm) 2.16 (3H, s), 2.24 (3H, s), 6.04 (1H, s), 7.30 (1H, d, J=8.4Hz), 7.59 (1H, dd, J=2.0, 8.4Hz), 7.64 (1H, d, J=2.0Hz).
MS (ESI); m/z 233 (M+H)⁺

### Preparation example 38

### 4-(3,5-Dimethyl-1H-pyrazol-1-yl)-3-hydroxy-N,N-dimethylbenzamide

36 mg of 4-(3,5-dimethyl-1H-pyrazol-1-yl)-3-hydroxybenzene carboxylic acid was dissolved in 0.4 ml of dimethylformamide, and 36 mg of 1-(3-dimethylaminopropyl)-3-ethylcarbodiimide hydrochloride, 25 mg of 1-hydroxybenzotriazole and 93 µl of dimethylamine (a 2.0M THF solution) were added, followed by stirring at room temperature for 4 hours. To the reaction mixture was added 10 ml of water, followed by extracting with 15 ml of ethyl acetate. The organic layer was dried over anhydrous magnesium sulfate, subsequently the solvent was distilled off under reduced pressure, and purification using preparative thin-layer silica gel column chromatography (ethyl acetate) afforded 11.4 mg of the title compound.
¹H-NMR (CDCl₃); δ (ppm) 2.31 (3H, s), 2.41 (3H, s), 3.02 (3H, s), 3.11 (3H, s), 6.06 (1H, s), 6.97 (1H, d, J=8.0Hz), 7.23 (1H, d, J=8.0Hz), 7.26 (1H, s).
MS (ESI); m/z 259 (M+H)⁺

### Preparation example 39

### 4-(3,5-Dimethyl-1H-pyrazol-1-yl)-3-hydroxybenzamide

34.3 mg of the title compound was afforded from 73.1 mg of 4-(3,5-dimethyl-1H-pyrazol-1-yl)-3-hydroxybenzene carboxylic acid and 38 µl of aqueous ammonia in a manner similar to Preparation example 38.
¹H-NMR (CD₃OD); δ (ppm) 2.16 (3H, s), 2.24 (3H, s), 6.04 (1H, s), 7.29 (1H, d, J=8.4Hz), 7.41 (1H, dd, J=2.0, 8.4Hz), 7.50 (1H, d, J=2.0Hz).
MS (ESI); m/z 232 (M+H)⁺

### Preparation example 40

### 3-Hydroxy-4-(3,4,5-trimethyl-1H-pyrazol-1-yl)benzene carboxylic acid

### a) Methyl 3-hydroxy-4-(3,4,5-trimethyl-1H-pyrazol-1-yl)-benzene carboxylate

110.4 mg of the title compound was afforded from 167 mg of methyl 4-amino-3-hydroxybenzenecarboxylate and 116 µl of 3-methyl-2,4-pentanedione in a manner similar to Preparation example 2b).
¹H-NMR (CDCl₃); δ (ppm) 2.00 (3H, s), 2.26 (3H, s), 2.36 (3H, s), 3.92 (3H, s), 7.23-7.26 (1H, m), 7.58-7.60 (1H, m), 7.49 (1H, s), 10.53 (1H, s).
MS (ESI); m/z 261 (M+H)⁺

### b) 3-Hydroxy-4-(3,4,5-trimethyl-1H-pyrazol-1-yl)benzene carboxylic acid

68.7 mg of the title compound was afforded from 110 mg of methyl 3-hydroxy-4-(3,4,5-trimethyl-1H-pyrazol-1-yl)-benzene carboxylate in a manner similar to Preparation example 37.
¹H-NMR (CD₃OD); δ (ppm) 1.99 (3H, s), 2.09 (3H, s), 2.20 (3H, s), 7.28 (1H, d, J=8.0Hz),7.59 (1H, dd, J=2.0, 8.0Hz), 7.63 (1H, d, J=2.0Hz).
MS (ESI); m/z 247 (M+H)⁺

### Preparation example 41

### 3-Hydroxy-4-(3,4,5-trimethyl-1H-pyrazol-1-yl)benzamide

33.3 mg of the title compound was afforded from 65 mg of 3-hydroxy-4-(3,4,5-trimethyl-1H-pyrazol-1-yl)benzene carboxylic acid and 32 µl of aqueous ammonia in a manner similar to Preparation example 38.
¹H-NMR (CD₃OD); δ (ppm) 1.99 (3H, s), 2.09 (3H, s), 2.19 (3H, s), 7.27 (1H, d, J=8.0Hz),7.40 (1H, dd, J=2.0, 8.0Hz), 7.57 (1H, d, J=2.0Hz).
MS (ESI); m/z 246 (M+H)⁺

### Preparation example 42

### 4-(4-Chloro-3,5-dimethyl-1H-pyrazol-1-yl)-3-hydroxybenzamide

### a) Methyl 4-(4-chloro 3,5-dimethyl-1H-pyrazol-1-yl)-3-hydroxybenzoate

69.1 mg of the title compound was afforded from 167 mg of methyl 4-amino-3-hydroxybenzoate and 114 µl of 3-chloropentane-2,4-dione in a manner similar to Preparation example 2b).
¹H-NMR (CDCl₃); δ (ppm) 2.32 (3H, s), 2.42 (3H, s), 3.93 (3H, s), 7.24-7.26 (1H, m), 7.60-7.66 (1H, m), 7.77 (1H, s).

### b) 4-(4-Chloro-3,5-dimethyl-1H-pyrazol-1-yl)-3-hydroxybenzoic acid

39.2 mg of the title compound was afforded from 6 9.1 mg of methyl 4-(4-chloro3,5-dimethyl-1H-pyrazol-1-yl)-3-hydroxybenzoate in a manner similar to Preparation example 37.
¹H-NMR (CD₃OD); δ (ppm) 2.15 (3H, s), 2.24 (3H, s), 7.32-7.35 (1H, m), 7.59-7.66 (2H, m).
MS (ESI); m/z 267 (M+H)⁺

### c) 4-(4-Chloro-3,5-dimethyl-1H-pyrazol-1-yl)-3-hydroxybenzamide

12.3 mg of the title compound was afforded from 37.9 mg of 4-(4-chloro-3,5-dimethyl-1H-pyrazol-1-yl)-3-hydroxybenzoic acid and 1 7 µl of aqueous ammonia in a manner similar to Preparation example 38.
¹H-NMR (CD₃OD); δ (ppm) 2.14 (3H, s), 2.24 (3H, s), 7.29-7.32 (1H, m), 7.41-7.44 (1H, m), 7.50 (1H, s).
MS (ESI); m/z 266 (M+H)⁺

### Preparation example 43 2-(3,5-Dimethyl-1H-pyrazol-1-yl)benzene-1,3-diol

### a) 2-Aminobenzene-1,3-diol

465 mg of 2-nitroresorcinol was dissolved in 9.3 ml of ethanol, 230 mg of 10% palladium/carbon was added, followed by stirring under a hydrogen atmosphere at room temperature for 1 hour. The insoluble material was filtered and subsequently the solvent was distilled off under reduced pressure to afford 338.5 mg of the title compound.
¹H-NMR (DMSO-d6); δ (ppm) 6.20-6.28 (3H, m).
MS (ESI); m/z 126 (M+H)⁺

### b) 2-Amino-1,3-phenylene bis(4-methylbenzenesulfonate)

100 mg of 2-aminobenzene-1,3-diol was dissolved in 2 ml of dichloromethane, 234 µl of triethylamine and 320 mg of p-toluenesulfonyl chloride were added, followed by stirring at room temperature for 1.5 hours. To the reaction mixture was added 20 ml of water, followed by extracting with 20 ml of ethyl acetate. The organic layer was dried over anhydrous magnesium sulfate, subsequently the solvent was distilled off under reduced pressure, and purification using silica gel column chromatography (hexane:ethyl acetate=4:1) afforded 278.1 mg of the title compound.
¹H-NMR (CDCl₃); δ (ppm) 2.46(6H, s), 3.83 (2H, s), 6.48 (1H, t, J=8.1Hz), 6.79 (2H, d, J=8.3Hz), 7.32(4H, d, J=8.1Hz), 7.72 (4H, d, J=8.3Hz).
MS (ESI); m/z 434 (M+H)⁺

### c) 2-(3,5-Dimethyl-1H-pyrazol-1-yl)-3-hydroxyphenyl 4-methylbenzenesulfonate

To 278 mg of 2-amino-1,3-phenylene bis(4-methylbenzenesulfonate) was added 0.64 ml of 5N-hydrochloric acid, and a solution of 58 mg of sodium nitrite dissolved in 0.4 ml of water was added dropwise at 0°C, followed by stirring for 30 minutes. Then, a solution of 289 mg of stannous chloride dissolved in 0.32 ml of 5N-hydrochloric acid was added dropwise at 0°C, followed by stirring for 1 hour. The solvent was distilled off under reduced pressure, and 1.3 ml of ethanol and 66 µl of acetylacetone were added, followed by heating to reflux for 2 hours. To the reaction mixture was added 50 ml of water, followed by neutralizing with saturated sodium hydrogen carbonate solution and extracting with 50 ml of ethyl acetate. The organic layer was dried over anhydrous magnesium sulfate, subsequently the solvent was distilled off under reduced pressure, and purification using silica gel column chromatography (hexane:ethyl acetate=4:1-1:1) afforded 62.9 mg of the title compound.
¹H-NMR (CDCl₃); δ (ppm) 2.15 (3H, s), 2.17 (3H, s), 2.41 (3H, s), 5.91 (1H, s), 6.93-6.-96 (2H, m), 7.14 (2H, d, J=8.0Hz), 7.18-7.26 (1H, m), 7.35 (2H, d, J=8.0Hz).
MS (ESI); m/z 359 (M+H)⁺

### d) 2-(3,5-Dimethyl-1H-pyrazol-1-yl)benzene-1,3-diol

To 62 mg of 2-(3,5-dimethyl-1H-pyrazol-1-yl)-3-hydroxyphenyl 4-methylbenzenesulfonate was added a solution of 97 mg of potassium hydroxide dissolved in 1.5 ml of ethanol and 1.5 ml of water, followed by heating to reflux for 3.5 hours. To the reaction mixture was added 20 ml of water, followed by extracting with 20 ml of ethyl acetate. The organic layer was dried over anhydrous magnesium sulfate, subsequently the solvent was distilled off under reduced pressure, and purification using preparative thin-layer silica gel column chromatography (hexane:ethyl acetate=1:1) afforded 20.8 mg of the title compound.
¹H-NMR (CDCl₃); δ (ppm) 2.15 (3H, s), 2.16 (3H, s), 5.98 (1H, s), 6.45 (2H, d, J=8.4Hz), 7.00 (1H, t, J=8.4Hz).
MS (ESI); m/z 205 (M+H)⁺

### Preparation example 44 2-(3,5-Dimethyl-1H-pyrazol-1-yl)-5-methylphenol

### a) 2-Amino-5-methylphenyl 4-methylbenzenesulfonate

400 mg of 2-amino-5-methylphenol was dissolved in 6.5 ml of dichloromethane, and 476 µl of triethylamine and 619 mg of p-toluenesulfonyl chloride were added, followed by stirring at room temperature for 1 hour. To the reaction mixture was added 60 ml of water, followed by extracting with 60 ml of ethyl acetate. The organic layer was dried over anhydrous magnesium sulfate, subsequently the solvent was distilled off under reduced pressure, and purification using silica gel column chromatography (hexane:ethyl acetate=3:1) afforded 730.1 mg of the title compound.
¹H-NMR (CDCl₃); δ (ppm) 2.15 (3H, s), 2.46 (3H, s), 3.63 (2H, brs), 6.61-6.63 (1H, m), 6.67 (1H, s), 6.82-6.85 (1H, m), 6.33 (2H, d, J=8.4Hz), 7.78 (2H, d, J=8.4Hz).MS (ESI); m/z 278 (M+H)⁺

### b) 2-(3,5-Dimethyl-1H-pyrazol-1-yl)-5-methylphenyl 4-methylbenzenesulfonate

To 453 mg of 2-amino-5-methylphenyl 4-methylbenzenesulfonate was added 1.6 ml of 5N-hydrochloric acid, and a solution of 146 mg of sodium nitrite dissolved in 1 ml of water was added dropwise at 0°C, followed by stirring for 30 minutes. Then, a solution of 736 mg of stannous chloride dissolved in 0.8 ml of 5N-hydrochloric acid was added dropwise at 0°C, followed by stirring for 1 hour. The solvent was distilled off under reduced pressure and 3.2 ml of ethanol and 167 µl of acetylacetone were added, followed by heating to reflux for 2 hours. To the reaction mixture was added 50 ml of water, followed by neutralizing with saturated sodium hydrogen carbonate solution and extracting with 80 ml of ethyl acetate. The organic layer was dried over anhydrous magnesium sulfate, subsequently the solvent was distilled off under reduced pressure, and purification using silica gel column chromatography (hexane:ethyl acetate=2:1) afforded 199.6 mg of the title compound.
¹H-NMR (CDCl₃); δ (ppm) 2.09 (3H, s), 2.11 (3H, s), 2.41 (3H,s), 2.43 (3H, s), 5.82 (1H, s), 7.15-7.21(4H, m), 7.36-7.38 (3H, m).
MS (ESI); m/z 357 (M+H)⁺

### c) 2-(3,5-Dimethyl-1H-pyrazol-1-yl)-5-methylphenol

To 199.6 mg of 2-(3,5-dimethyl-1H-pyrazol-1-yl)-5-methylphenyl 4-methylbenzenesulfonate was added a solution of 314 mg of potassium hydroxide dissolved in 4 ml of ethanol and 4 ml of water, followed by heating to reflux for 1 hour. To the reaction mixture was added 20 ml of water, followed by extracting with 20 ml of ethyl acetate. The organic layer was dried over anhydrous magnesium sulfate, subsequently the solvent was distilled off under reduced pressure, purification was carried out using preparative thin-layer silica gel column chromatography (hexane:ethyl acetate=4:1), the desired fraction was dissolved in 1,4-dioxane, and subsequently lyophilization afforded 66.2 mg of the title compound.
¹H-NMR (CDCl₃); δ (ppm) 2.29 (3H, s), 2.33 (3H,s), 2.37 (3H,s), 6.01 (1H, s), 6.70(1H, d, J=8.0Hz), 6.90 (1H, s), 7.07 (1H, d, J=8.0Hz), 9.64 (1H, brs).
MS (ESI); m/z 203 (M+H)⁺

### Preparation example 45 2-(3,5-Dimethyl-1H-pyrazol-1-yl)-5-methoxyphenol

### a) 2-Amino-5-methoxyphenyl 4-methylbenzenesulfonate

220.1 mg of the title compound was afforded from 176 mg of 2-hydroxy-4-methoxyaniline hydrochloride in a manner similar to Preparation example 44 a).
¹H-NMR (CDCl₃); δ (ppm) 2.46 (3H, s), 3.62 (3H, s), 6.41 (1H, s), 6.65-6.66 (2H, m), 7.34 (2H, d, J=8.8Hz), 7.79 (2H, d, J=8.8Hz).

### b) 2-(3,5-Dimethyl-1H-pyrazol-1-yl)-5-methoxyphenyl 4-methylbenzenesulfonate

93.7 mg of the title compound was afforded from 220 mg of 2-amino-5-methoxyphenyl 4-methylbenzenesulfonate in a manner similar to Preparation example 44b).
¹H-NMR (CDCl₃); δ (ppm) 2.08 (3H, s), 2.11 (3H, s), 2.41 (3H, s), 3.85 (3H, s), 5.82 (1H, s), 6.87 (1H, dd, J=2.4, 8.4Hz), 7.06 (1H, d, J=2.4Hz), 7.17 (2H, d, J=8.4Hz), 7.20-7.24 (1H, m), 7.39 (2H, d, J=8.4Hz).
MS (ESI); m/z 373 (M+H)⁺

### c) 2-(3,5-Dimethyl-1H-pyrazol-1-yl)-5-methoxyphenol

30.1 mg of the title compound was afforded from 93 mg of 2-(3,5-dimethyl-1H-pyrazol-1-yl)-5-methoxyphenyl 4-methylbenzenesulfonate in a manner similar to Preparation example 44c).
¹H-NMR (CDCl₃); δ (ppm) 2.27 (3H, s), 2.33 (3H, s), 3.80 (3H, s), 6.00 (1H, s), 6.45 (1H, dd, J=2.8, 8.4Hz), 6.61 (1H, d, J=2.8Hz), 7.08 (1H, d, J=8.4Hz), 9.67 (1H, brs).
MS (ESI); m/z 219 (M+H)⁺

### Preparation example 46 2-(3,5-Dimethyl-1H-pyrazol-1-yl)-3-methylphenol

### a) 2-Amino-3-methylphenyl 4-methylbenzenesulfonate

374.1 mg of the title compound was afforded from 200 mg of 2-amino-3-methylphenol in a manner similar to Preparation example 43b).
¹H-NMR (CDCl₃); δ (ppm) 2.14 (3H, s), 2.46 (3H, s), 3.79 (2H,s), 6.51 (1H, t, J=8.0Hz), 6.63 (1H, d, J=8.0Hz), 6.91 (1H, d, J=8.0Hz), 7.32 (2H, d, J=8.4Hz), 7.78 (2H, d, J=8.4Hz).
MS (ESI); m/z 278 (M+H)⁺

### b) 2-(3,5-Dimethyl-1H-pyrazol-1-yl)-3-methylphenyl 4-methylbenzenesulfonate

269.9 mg of the title compound was afforded from 374 mg of 2-amino-3-methylphenyl 4-methylbenzenesulfonate in a manner similar to Preparation example 43c).
¹H-NMR (CDCl₃); δ (ppm) 2.01 (3H, s), 2.02 (3H, s), 2.16 (3H, s), 2.43 (3H, s), 5.88 (1H, s), 7.19-7.24 (3H, m), 7.32 (2H, d, J=5.2Hz), 7.51 (2H, d, J=8.4Hz).
MS (ESI); m/z 357 (M+H)⁺

### c) 2-(3,5-Dimethyl-1H-pyrazol-1-yl)-3-methylphenol

79.2 mg of the title compound was afforded from 269 mg of 2-(3,5-dimethyl-1H-pyrazol-1-yl)-3-methylphenyl 4-methylbenzenesulfonate in a manner similar to Preparation example 43d).
¹H-NMR (CD₃OD); δ (ppm) 1.92 (3H, s), 2.01 (3H, s), 2.24 (3H, s), 6.02 (1H, s), 6.79 (2H, d, J=7.6Hz), 7.18 (1H, t, J=7.6Hz).
MS (ESI); m/z 203 (M+H)⁺

### Preparation example 47

### 2-(3,5-Dimethyl-1H-pyrazol-1-yl)-5-hydroxymethylphenol

132 mg of methyl 4-(3,5-dimethyl-1H-pyrazol-1-yl)-3-hydroxybenzenecarboxylate was dissolved in 2.6 ml of tetrahydrofuran and 58 mg of lithium borohydride was added, followed by stirring at 50°C for 3.5 hours. To the reaction mixture was added 20 ml of water, followed by extracting with 20 ml of ethyl acetate. The organic layer was dried over anhydrous magnesium sulfate, subsequently the solvent was distilled off under reduced pressure, and purification using preparative thin-layer silica gel column chromatography (hexane:ethyl acetate=1:2) afforded 12.4 mg of the title compound.
¹H-NMR (CDCl₃); δ (ppm) 2.28 (3H,s), 2.44 (3H, s), 4.64 (2H, s), 6.02 (1H, s), 6.90 (1H, d, J=8.0Hz), 7.02 (1H, s), 7.17 (1H, d, J=8.0Hz).
MS (ESI); m/z 219 (M+H)⁺

### Preparation example 48

### 2-(3,5-Dimethyl-1H-pyrazol-1-yl)-5-methylaminophenol

### a) 1-(2-Methoxy-4-nitrophenyl)-3,5-dimethyl-1H-pyrazole

498 mg of the title compound was afforded from 700 mg of 2-methoxy-4-nitroaniline in a manner similar to Preparation example 43c).
¹H-NMR (CDCl₃); δ (ppm) 2.13 (3H, s), 2.30 (3H, s), 3.93 (3H, s), 6.02 (1H, s), 7.53 (1H, d, J=8.4Hz), 7.89 (1H, d, J=2.0Hz), 7.95 (1H, dd, J=2.0, 8.4Hz).
MS (ESI); m/z 248 (M+H)⁺

### b) 4-(3,5-Dimethyl-1H-pyrazol-1-yl)-3-methoxyaniline

417.6 mg of the title compound was afforded from 495 mg of 1-(2-methoxy-4-nitrophenyl)-3,5-dimethyl-1H-pyrazole in a manner similar to Preparation example 31.
¹H-NMR (CDCl₃); δ (ppm) 2.06 (3H, s), 2.28 (3H, s), 3.72 (3H, s), 3.81 (2H, brs), 5.92 (1H, s), 6.28-6.31 (2H, m), 7.06 (1H, d, J=8.4Hz).
MS (ESI); m/z 218 (M+H)⁺

### c) 4-(3,5-Dimethyl-1H-pyrazol-1-yl)-3-methoxy-N-methylaniline

200 mg of 4-(3,5-dimethyl-1H-pyrazol-1-yl)-3-methoxyaniline was dissolved in 6 ml of dimethylformamide, and 160 µl of methyl iodide and 636 mg of potassium carbonate were added, followed by stirring at room temperature for 2.5 hours. To the reaction mixture was added 50 ml of water, followed by extracting with 50 ml of ethyl acetate. The organic layer was dried over anhydrous magnesium sulfate, subsequently the solvent was distilled off under reduced pressure, and purification using preparative thin-layer silica gel column chromatography (hexane:ethyl acetate=1:3) afforded 48.7 mg of the title compound.
¹H-NMR (CDCl₃); δ (ppm) 2.07 (3H, s), 2.28 (3H, s), 2.86 (3H, s), 3.74 (3H, s), 3.95 (1H, brs), 5.92 (1H, s), 6.17 (1H, d, J=2.4Hz), 6.21 (1H, dd, J=2.4, 8.4Hz), 7.09 (1H, d, J=8.4Hz).
MS (ESI); m/z 232 (M+H)⁺

### d) 2-(3,5-Dimethyl-1H-pyrazol-1-yl)-5-methylaminophenol

25 mg of the title compound was afforded from 58.7 mg of 4-(3,5-dimethyl-1H-pyrazol-1-yl)-3-methoxy-N-methylaniline in a manner similar to Preparation example 33c).
¹H-NMR (CDCl₃); δ (ppm) 2.28 (3H, s), 2.32 (3H, s), 2.83 (3H, d, J=1.2Hz), 5.98 (1H, s), 6.13-6.16 (1H, m), 6.31-6.32 (1H, m), 6.99 (1H, dd, J=1.2, 8.4Hz).
MS (ESI); m/z 218 (M+H)⁺

### Preparation example 49 2-(3,5-Dimethyl-1H-pyrazol-1-yl)-4-methylphenol

### a) 2-Amino-4-methylphenyl 4-methylbenzenesulfonate

770.1 mg of the title compound was afforded from 479 mg of 2-amino-4-methylphenol hydrochloride in a manner similar to Preparation example 43b).
¹H-NMR (CDCl₃); δ (ppm) 2.21 (3H, s), 2.46 (3H, s), 3.74 (2H, brs), 6.39 (1H, d, J=8.0Hz), 6.53 (1H, s), 6.62 (1H, d, J=8.0Hz), 7.32 (2H, d, J=8.0Hz), 7.77 (2H, d, J=8.0Hz).
MS (ESI); m/z 278 (M+H)⁺

### b) 2-(3,5-Dimethyl-1H-pyrazol-1-yl)-4-methylphenyl 4-methylbenzenesulfonate

137.3 mg of the title compound was afforded from 400 mg of 2-amino-4-methylphenyl 4-methylbenzenesulfonate in a manner similar to Preparation example 43c).
¹H-NMR (CDCl₃); δ (ppm) 2.10 (3H, s), 2.12 (3H, s), 2.35 (3H, s), 2.41 (3H, s), 5.82 (1H, s), 7.13-7.15 (3H, m), 7.20-7.22 (1H, m), 7.33-7.36 (2H, m), 7.42 (2H, d, J=8.4Hz).
MS (ESI); m/z 357 (M+H)⁺

### c) 2-(3,5-Dimethyl-1H-pyrazol-1-yl)-4-methylphenol

60.4 mg of the title compound was afforded from 167 mg of 2-(3,5-dimethyl-1H-pyrazol-1-yl)-4-methylphenyl 4-methylbenzenesulfonate in a manner similar to Preparation example 43d).
¹H-NMR (CDCl₃); δ (ppm) 2.29 (3H, s), 2.31 (3H, s), 2.39 (3H, s), 6.02 (1H, s), 6.97-7.02 (3H, m), 9.43 (1H, s).
MS (ESI); m/z 203 (M+H)⁺

### Preparation example 50

### 2-(3,5-Dimethyl-1H-pyrazol-1-yl)-5-trifluoromethylphenol

### a) 1-(2-Methoxy-4-(trifluoromethylphenyl)-3,5-dimethyl-1H-pyrazole

95.5 mg of the title compound was afforded from 191 mg of 2-methoxy-4-trifluoromethylaniline in a manner similar to Preparation example 43c).
¹H-NMR (CDCl₃); δ (ppm) 2.05 (3H,s), 2.30 (3H, s), 3.86 (3H, s), 5.99 (1H,s), 7.22 (1H,s), 7.31 (1H, d, J=8.0Hz), 7.46 (1H, d, J=8.0Hz).
MS (ESI); m/z 271 (M+H)⁺

### b) 2-(3,5-Dimethyl-1H-pyrazol-1-yl)-5-trifluoromethylphenol

25.7 mg of the title compound was afforded from 95.5 mg of 1-(2-methoxy-4-trifluoromethylphenyl)-3,5-dimethyl-1H-pyrazole in a manner similar to Preparation example 33c).
¹H-NMR (CDCl₃); δ (ppm) 2.31 (3H, s), 2.45 (3H, s), 6.08 (1H,s), 7.15-7.18 (1H, m), 7.30-7.35 (2H, m), 10.64 (1H,s).
MS (ESI); m/z 257 (M+H)⁺

### Preparation example 51 2-(3,5-Dimethyl-1H-pyrazol-1-yl)-6-methylphenol

### a) 2-Amino-6-methylphenyl 4-methylbenzenesulfonate

159.9 mg of the title compound was afforded from 200 mg of 6-amino-o-cresol hydrochloride in a manner similar to Preparation example 43b).
¹H-NMR (CDCl₃); δ (ppm) 2.06 (3H, s), 2.48 (3H, s), 3.96 (2H, s), 6.53-6.55 (1H, m), 6.59-6.61 (1H, m), 6.93 (1H, t, J=7.6Hz), 7.37 (2H, d, J=8.0Hz), 7.90 (2H, d, J=8.0Hz).
MS (ESI); m/z 278 (M+H)⁺

### b) 2-(3,5-Dimethyl-1H-pyrazol-1-yl)-6-methylphenyl 4-methylbenzenesulfonate

48.8 mg of the title compound was afforded from 159.5 mg of 2-amino-6-methylphenyl 4-methylbenzenesulfonate in a manner similar to Preparation example 43c).
¹H-NMR (CDCl₃); δ (ppm) 2.09 (3H, s), 2.10 (3H, s), 2.43 (3H, s), 2.49 (3H, s), 5.67 (1H, s), 7.13-7.32 (5H, m), 7.48 (2H, d, J=8.0Hz).
MS (ESI); m/z 357 (M+H)⁺

### c) 2-(3,5-Dimethyl-1H-pyrazol-1-yl)-6-methylphenol

12 mg of the title compound was afforded from 48.5 mg of 2-(3,5-dimethyl-1H-pyrazol-1-yl)-6-methylphenyl 4-methylbenzenesulfonate in a manner similar to Preparation example 43d).
¹H-NMR (CDCl₃); δ (ppm) 2.30 (3H, s), 2.31 (3H, s), 2.38 (3H, s), 6.03 (1H, s), 6.81 (1H, t, J=8.0Hz), 7.03-7.09 (2H, m), 9.79 (1H, s).
MS (ESI); m/z 203 (M+H)⁺

### Preparation example 52 2-(3,5-Dimethyl-1H-pyrazol-1-yl)-5-ethylphenol

### a) 4-Chloro-5-ethyl-2-nitrophenyl methanesulfonate

150 mg of 4-chloro-5-ethyl-2-nitrophenol was dissolved in 1.5 ml of dichloromethane, and 156 µl of triethylamine and 69 µl of methanesulfonyl chloride were added, followed by stirring at room temperature for 30 minutes. To the reaction mixture was added 50 ml of water, followed by extracting with 60 ml of ethyl acetate. The organic layer was dried over anhydrous magnesium sulfate and subsequently the solvent was distilled off under reduced pressure to afford 200.7 mg of the title compound.
¹H-NMR (CDCl₃); δ (ppm) 1.28 (3H, t, J=7.6Hz), 2.83 (2H, q, J=7.6Hz), 3.37 (3H, s), 7.42 (1H, s), 8.09 (1H, s).

### b) 2-Amino-5-ethylphenyl methanesulfonate

200 mg of 4-chloro-5-ethyl-2-nitrophenyl methanesulfonate was dissolved in 4 ml of ethanol and 200 mg of 10% palladium/carbon was added, followed by stirring under a hydrogen atmosphere at room temperature for 4 hours. The insoluble material was filtered and subsequently the solvent was distilled off under reduced pressure to afford 74.9 mg of the title compound.
¹H-NMR (CD₃OD); δ (ppm) 1.26 (3H, t, J=7.6Hz), 2.73 (2H,q, J=7.6Hz), 3.47 (3H, s), 7.28-7.46 (3H, m).
MS (ESI); m/z 216 (M+H)⁺

### c) 2-(3,5-Dimethyl-1H-pyrazol-1-yl)-5-ethylphenyl methanesulfonate

28.1 mg of the title compound was afforded from 74.5 mg of 2-amino-5-ethylphenyl methanesulfonate in a manner similar to Preparation example 43c).
¹H-NMR (CDCl₃); δ (ppm) 1.28 (3H, t, J=7.6Hz), 2.17 (3H, s), 2.26 (3H, s), 2.65 (3H, s), 2.73 (2H, q, J=7.6Hz), 6.00 (1H, s), 7.24-7.26 (1H, m), 7.33-7.37 (2H, m).
MS (ESI); m/z 295 (M+H)⁺

### d) 2-(3,5-Dimethyl-1H-pyrazol-1-yl)-5-ethylphenol

28 mg of 2-(3,5-dimethyl-1H-pyrazol-1-yl)-5-ethylphenyl methanesulfonate was dissolved in 0.1 ml of methanol and 0.07 ml of 5N-hydrochloric acid was added, followed by heating to reflux for 30 minutes. To the reaction mixture was added 15 ml of water, followed by extracting with 15 ml of ethyl acetate. The organic layer was dried over anhydrous magnesium sulfate, subsequently the solvent was distilled off under reduced pressure, and purification using preparative thin-layer silica gel column chromatography (hexane:ethyl acetate=4:1) afforded 7.4 mg of the title compound.
¹H-NMR (CDCl₃); δ (ppm) 1.24 (3H, t, J=7.6Hz), 2.29 (3H, s), 2.38 (3H, s), 2.63 (2H, q, J=7.6Hz), 6.02 (1H, s), 6.73-6.75 (1H, m), 6.94 (1H, s), 7.10 (1H, d, J=8.0Hz), 9.67 (1H, s).
MS (ESI); m/z 217 (M+H)⁺

### Preparation example 53 2-(4-Fluoro-3,5-dimethyl-1H-pyrazol-1-yl)phenol

### a) 1-(2-Benzyloxyphenyl)-4-fluoro-3,5-dimethyl-1H-pyrazole

500 mg of 2-benzyloxyphenylhydrazine hydrochloride and 251 mg of 3-fluoropentane-2,4-dione were added to 12 ml of ethanol, followed by heating to reflux for 1.5 hours. The reaction solution was concentrated under reduced pressure as such, and purification using silica gel column chromatography (hexane:ethyl acetate=6:1-5:1) afforded 417 mg of the title compound.
¹H-NMR (CDCl₃); δ (ppm) 2.07 (3H, s), 2.30 (3H, s), 5.04 (2H, s), 7.03-7.07 (2H, m), 7.24-7.37 (7H, m).
MS (ESI); m/z 297 (M+H)⁺

### b) 2-(4-Fluoro-3,5-dimethyl-1H-pyrazol-1-yl)phenol

416 mg of 1-(2-benzyloxyphenyl)-4-fluoro-3,5-dimethyl-1H-pyrazole was dissolved in 16 ml of methanol and 42.6 mg of 10% palladium/carbon was added, followed by stirring under a hydrogen atmosphere at room temperature for 24 hours. The insoluble material was filtered, subsequently the filtrate was distilled off under reduced pressure, and the resulting residue was purified using silica gel column chromatography (hexane:ethyl acetate=5:1) to give 290 mg of the title compound.
¹H-NMR (CDCl₃); δ (ppm) 2.28 (3H, s), 2.33 (3H, s), 6.90 (1H, t, J=8.0Hz), 7.06 (1H, d, J=8.0Hz), 7.15 (1H, d, J=8.0Hz), 7.19 (1H, t, J=8.0Hz).
MS (ESI); m/z 207 (M+H)⁺

### Preparation example 54 5-Bromo-2-(3,5-dimethyl-1H-pyrazol-1-yl)phenol

### a) 5-Bromo-2-hydrazinylphenol 4-methylbenzenesulfonate

1.0 g of 2-amino-5-bromophenol was suspended in 7 ml of ethanol and 1.5 ml of concentrated hydrochloric acid was added dropwise at -10°C. Furthermore, 636 mg of tert-butyl nitrite was added dropwise at the same temperature, followed by stirring at the same temperature for 1 hour to afford a diazonium salt solution. Another flask was charged with 2.49 g of stannous chloride dihydrate, 1.08 g of p-toluenesulfonic acid monohydrate and 15 ml of ethanol, followed by stirring at -10°C for 15 minutes. Into this solution was added dropwise the above prepared diazonium salt solution at -10°C, followed by stirring at the same temperature for 1 hour. 30 ml of tert-butyl methylether was added, followed by stirring for 15 minutes. Subsequently, the resulting precipitate was filtered off to afford 0.9 g of the title compound.

### b) 5-Bromo-2-(3,5-dimethyl-1H-pyrazol-1-yl)phenol

0.9 g of 5-bromo-2-hydrazinylphenol 4-methylbenzenesulfonate and 0.8 g of acetylacetone were added into 25 ml of ethanol, followed by heating to reflux for 1 hour. The reaction was cooled to room temperature and concentrated under reduced pressure as such, and the resulting residue was dissolved in 50 ml of ethyl acetate, followed by washing twice with 20 ml of saturated aqueous sodium bicarbonate. The organic layer was dehydrated with anhydrous sodium sulphate and subsequently concentrated under reduced pressure, and the resulting residue was purified by silica gel column chromatography (hexane:ethyl acetate=4:1) to afford 650 mg of the title compound.
1H-NMR (CDCl₃); δ (ppm) 2.30 (3H, s), 2.40 (3H, s), 6.05 (1H, s), 7.01-7.08 (2H, m), 7.26 (1H, s).
MS (ESI); m/z 267 (M+H)⁺

### Preparation example 55

### 5-Bromo-2-(4-chloro-3,5-dimethyl-1H-pyrazol-1-yl)phenol

558 mg of the title compound was afforded from 0.9 g of 5-bromo-2-hydrazinylphenol 4-methylbenzenesulfonate prepared in analogy to Preparation example 54a) and 1.07 g of 3-chloropentane-2,4-dione, in a manner similar to Preparation example 54b).
1H-NMR (CDCl₃); δ (ppm) 2.30 (3H, s), 2.38 (3H, s), 7.02-7.08 (2H, m), 7.27 (1H, d, J=2.0Hz), 9.62 (1H, s).
MS (ESI); m/z 303 (M+H)⁺

### Preparation example 56 5-Bromo-2-(3,4,5-trimethyl-1H-pyrazol-1-yl)phenol

320 mg of the title compound was afforded from 0.9 g of 5-bromo-2-hydrazinylphenol 4-methylbenzenesulfonate prepared in analogy to Preparation example 54a) and 0.9 g of 3-methylpentane-2,4-dione, in a manner similar to Preparation example 54b).
1H-NMR (CDCl₃); δ (ppm) 1.98 (3H, s), 2.24 (3H, s), 2.31 (3H, s), 7.00-7.06 (2H, m), 7.25 (1H, d, J=1.6 Hz)), 10.34 (1H, s).
MS (ESI); m/z 281 (M+H)⁺

### Preparation example 57 4-(3,5-Dimethyl-1H-pyrazol-1-yl)-3-hydroxyphenyl acetate

### a) 4-Nitro-1,3-phenylene diacetate

Under a nitrogen atmosphere, 5.0 g of 4-nitrobenzene-1,3-diol was dissolved in 50 ml of methylene chloride, and under ice cooling, 5.35 g of pyridine, 0.39 g of 4-dimethylaminopyridine and 8.12 g of acetic anhydride were sequentially added. The temperature of the reaction was raised to room temperature, followed by stirring for 1 hour. The reaction solution was washed with 50 ml of water, 100 ml of 1N-hydrochloric acid, 100 ml of saturated aqueous sodium bicarbonate, and 100 ml of saturated brine sequentially, dehydrated with anhydrous sodium sulphate, and subsequently concentrated under reduced pressure to afford 7.4 g of the title compound. 1H-NMR (CDCl₃); δ (ppm) 2.33 (3H, s), 2.37 (3H, s), 7.09 (1H, d, J=2.4 Hz), 7.18 (1H, dd, J=2.4, 9.2 Hz), 8.16 (1H, d, J=9.2 Hz).

### b) 3-Hydroxy-4-nitrophenyl acetate

Under a nitrogen atmosphere, 1.0 g of 4-nitro-1,3-phenylene diacetate was dissolved in 25 ml of chloroform, and under ice cooling, 2.23 g of aluminium chloride was added. The temperature of the reaction was raised to room temperature, followed by stirring for 3 hours. 100 ml of water was added, followed by extracting three times with 30 ml of methylene chloride. The organic layers were combined, washed with 25 ml of 1N-hydrochloric acid and 25 ml of saturated brine sequentially, dehydrated with anhydrous sodium sulphate, and subsequently concentrated under reduced pressure. The resulting residue was purified by silica gel column chromatography (hexane:ethyl acetate=20:1) to afford 610 mg of the title compound.
1H-NMR (CDCl₃): δ (ppm) 2.32 (3H, s), 6.77 (1H, dd, J=2.4 Hz, J=9.2 Hz), 6.95 (1H, d, J=2.4 Hz), 8.14 (1H, d, J=9.2 Hz), 10.70 (1H, s).
MS (ESI); m/z 196(M-H)⁻

### c) 4-Amino-3-hydroxyphenyl acetate

3.0 g of 3-hydroxy-4-nitrophenyl acetate was dissolved in 50 ml of ethyl acetate and 300 mg of 10% palladium/carbon was added, followed by stirring under a hydrogen atmosphere at room temperature for 10 hours. The insoluble material was filtered, subsequently the filtrate was distilled off under reduced pressure, and the resulting residue was purified by silica gel column chromatography (hexane:ethyl acetate=1:1) to afford 2.48 g of the title compound.
1H-NMR (DMSO-d6); δ (ppm) 2.17 (3H, s), 4.45 (2H, brs), 6.28 (1H, dd, J=2.4, 8.4Hz), 6.40 (1H, d, J=2.4Hz), 6.53 (1H, d, J=8.4Hz), 9.26 (1H, brs).
MS (ESI); m/z 167 (M+)

### d) 4-(3,5-Dimethyl-1H-pyrazol-1-yl)-3-hydroxyphenyl acetate

330 mg of the title compound was afforded from 0.9 g of 4-hydrazinyl-3-hydroxyphenyl acetate 4-methylbenzenesulfonate prepared using 1.0 g of 4-amino-3-hydroxyphenyl acetate in analogy to Preparation example 54a) and 0.9 g of acetylacetone, in a manner similar to Preparation example 54b).
1H-NMR (CDCl₃); δ (ppm) 2.29 (3H, s), 2.30 (3H, s), 2.41 (3H, s), 6.04 (1H, s), 6.68 (1H, dd, J=2.4, 8.8Hz), 6.85 (1H, d, J=2.4Hz), 7.19 (1H, d, J=8.8 Hz), 10.14 (1H, s).
MS (ESI); m/z 247 (M+H)⁺

### Preparation example 58

### 4-(4-Chloro-3,5-dimethyl-1H-pyrazol-1-yl)-3-hydroxyphenyl acetate

30 mg of the title compound was afforded from 0.9 g of 4-hydrazinyl-3-hydroxyphenyl acetate 4-methylbenzenesulfonate prepared using 1.0 g of 4-amino-3-hydroxyphenyl acetate in analogy to Preparation example 54a) and 1.2 g of 3-chloropentane-2,4-dione, in a manner similar to Preparation example 54b).
1H-NMR (CDCl₃); δ (ppm) 2.30 (3H, s), 2.31 (3H, s), 2.39 (3H, s), 6.70 (1H, dd, J=2.8, 8.8Hz), 6.86 (1H, d, J=2.8Hz), 7.17 (1H, d, J=8.8 Hz), 9.50 (1H, s).
MS (ESI); m/z 281 (M+H)⁺

### Preparation example 59

### 3-Hydroxy-4-(3,4,5-trimethyl-1H-pyrazol-1-yl)phenyl acetate

230 mg of the title compound was afforded from 0.6 g of 4-hydrazinyl-3-hydroxyphenyl acetate 4-methylbenzenesulfonate prepared using 1.0 g of 4-amino-3-hydroxyphenyl acetate in analogy to Preparation example 54a) and 1.0 g of 3-methylpentane-2,4-dione, in a manner similar to Preparation example 54b).
1H-NMR (CDCl₃); δ (ppm) 1.99 (3H, s), 2.24 (3H, s), 2.30 (3H, s), 2.32 (3H, s), 6.66 (1H, dd, J=2.8, 8.4Hz), 6.83 (1H, d, J=2.8Hz), 7.17 (1H, d, J=8.4 Hz), 10.22 (1H, s).
MS (ESI); m/z 261 (M+H)⁺

### Preparation example 60

### 2-(3,5-Dimethyl-1H-pyrazol-1-yl)-4-methoxy-5-methylphenol

### a) 1,4-Dimethoxy-2-methyl-5-nitrobenzene

6.0 g of 2,5-dimethoxytoluene was dissolved in 20 ml of acetic acid, and at 40°C, a solution of 4.32 g of fuming nitric acid (d=1.50) in 10 ml of acetic acid was added dropwise over 5 minutes. The reaction was stirred at the same temperature for 30 minutes, cooled to room temperature, and subsequently stirred for another 30 minutes. The reaction solution was diluted with 300 ml of cold water, and the resulting precipitate was filtering off and washed with 100 ml of cold water. Drying under reduced pressure gave 7.5 g of the title compound.
1H-NMR (CDCl₃); δ (ppm) 2.28 (3H, s), 3.84 (3H, s), 3.92 (3H, s), 6.90 (1H, s), 7.40 (1H, s).
MS (ESI); m/z 198 (M+H)⁺

### b) 4-Methoxy-5-methyl-2-nitrophenol

A solution of 6.0 g of 1,4-dimethoxy-2-methyl-5-nitrobenzene in 30 ml of methylene chloride was cooled to -20°C and 30 ml of 1M solution of boron trichloride in methylene chloride was added dropwise at the same temperature. The temperature of the reaction was raised to room temperature and subsequently the reaction solution was stirred for 16 hours and added to 50 ml of saturated aqueous sodium bicarbonate, followed by extracting three times with 100 ml of ethyl acetate. The organic layers were combined, washed with 100 ml of water and 50 ml of saturated brine, subsequently dehydrated with anhydrous sodium sulphate, and concentrated under reduced pressure. The resulting residue was purified by silica gel column chromatography (hexane:ethyl acetate=9:1) to afford 4.25 g of the title compound.
1H-NMR (CDCl₃); δ (ppm) 2.26 (3H, s), 3.84 (3H, s), 6.94 (1H, s), 7.39 (1H, s), 10.46 (1H, s).
MS (ESI); m/z 182(M-H)⁻

### c) 4-Methoxy-5-methyl-2-nitrophenyl 4-methylbenzenesulfonate

Under a nitrogen atmosphere, to a solution of 8.0 g of 4-methoxy-5-methyl-2-nitrophenol in 80 ml of methylene chloride was added 9.15 g of p-toluenesulfonyl chloride at room temperature, followed by cooling to 0°C. 4.86 g of triethylamine was added thereto, followed by stirring for 2 hours. The reaction solution was poured to 150 ml of water, followed by extracting with 150 ml of methylene chloride. The organic layer was washed with 100 ml of water and 50 ml of saturated brine, dehydrated with anhydrous sodium sulphate, and subsequently concentrated under reduced pressure. The resulting residue was purified by silica gel column chromatography (hexane:ethyl acetate=9:1) to afford 12.0 g of the title compound.
1H-NMR (CDCl₃); δ (ppm) 2.26 (3H, s), 2.46 (3H, s), 3.87 (3H, s), 7.19 (1H, s), 7.33 (1H, s), 7.34 (2H, d, J=8.4Hz), 7.77 (2H, d, J=8.4Hz).
MS (ESI); m/z 336 (M-H)⁻

### d) 2-Amino-4-methoxy-5-methylphenyl 4-methylbenzenesulfonate

3.2 g of the title compound was afforded in a manner similar to Preparation example 57c) using 4.0 g of 4-methoxy-5-methyl-2-nitrophenyl 4-methylbenzenesulfonate.
1H-NMR (DMSO-d₆); δ (ppm) 1.91 (3H, s), 2.41 (3H, s), 3.64 (3H, s), 4.73 (2H, brs), 6.25 (1H, s), 6.64 (1H, s), 7.43 (2H, d, J=8.4Hz), 7.79 (2H, d, J=8.4Hz).
MS (ESI); m/z 308 (M+H)⁺

### e) 2-(3,5-Dimethyl-1H-pyrazol-1-yl)-4-methoxy-5-methylphenyl 4-methylbenzenesulfonate

400 mg of the title compound was afforded in a manner similar to Preparation examples 54a) and 54b) using 1.0 g of 2-amino-4-methoxy-5-methylphenyl 4-methylbenzenesulfonate.
1H-NMR (CDCl₃); δ (ppm) 2.10(6H, s), 2.25 (3H, s), 2.41 (3H, s), 3.79 (3H, s), 5.80 (1H, s), 6.71 (1H, s), 7.15 (2H, d, J=8.4Hz), 7.27 (1H, s), 7.35 (2H, d, J=8.4Hz).
MS (ESI); m/z 387 (M+H)⁺

### f) 2-(3,5-Dimethyl-1H-pyrazol-1-yl)-4-methoxy-5-methylphenol

70 mg of the title compound was afforded in a manner similar to Preparation example 29 using 200 mg of 2-(3,5-dimethyl-1H-pyrazol-1-yl)-4-methoxy-5-methylphenyl 4-methylbenzenesulfonate.
1H-NMR (CDCl₃); δ (ppm) 2.21 (3H, s), 2.29 (3H, s), 2.39 (3H, s), 3.78 (3H, s), 6.02 (1H, s), 6.67 (1H, s), 6.89 (1H, s), 8.87 (1H, s).
MS (ESI); m/z 233 (M+H)⁺

### Preparation example 61

### 4-Chloro-2-(3,5-dimethyl-1H-pyrazol-1-yl)-5-methylphenol

### a) 2-Amino-4-chloro-5-methylphenol

Into a solution of 5.0 g of 4-chloro-5-methyl-2-nitrophenol in 100 ml of methanol were added 8.71 g of zinc dust activated by hydrochloric acid and a solution of 7.1 g of ammonium chloride in 20 ml of water at 0°C. This suspension was stirred at room temperature for 4 hours and subsequently the insoluble material was filtered through Celite, followed by washing with 100 ml of ethyl acetate. The filtrate and washings were combined and concentrated under reduced pressure. The resulting residue was purified by silica gel column chromatography (hexane:ethyl acetate=3:2) to afford 1.4 g of the title compound.
1H-NMR (DMSO-d₆); δ (ppm) 2.10 (3H, s), 4.57 (2H, brs), 6.54 (1H, s), 6.58 (1H, s), 9.11 (1H, s).
MS (ESI); m/z 157(M)⁺

### b) 4-Chloro-2-(3,5-dimethyl-1H-pyrazol-1-yl)-5-methylphenol

120 mg of the title compound was afforded using 1.0 g of 2-amino-4-chloro-5-methylphenol in a manner similar to Preparation examples 54a) and 54b).
1H-NMR (CDCl₃); δ (ppm) 2.29 (3H, s), 2.34 (3H, s), 2.41 (3H, s), 6.03 (1H, s), 6.96 (1H, s), 7.19 (1H, s), 9.88 (1H, s).
MS (ESI); m/z 237 (M+H)⁺

### Preparation example 62

### 2-(3,5-Dimethyl-1H-pyrazol-1-yl)-4,5-dimethylphenol

630 mg of the title compound was afforded using 1.0 g of 2-amino-4,5-dimethylphenol in a manner similar to Preparation examples 54a) and 54b).
1H-NMR (CDCl₃); δ (ppm) 2.21 (3H, s), 2.24 (3H, s), 2.29 (3H, s), 2.38 (3H, s), 6.00 (1H, s), 6.88 (1H, s), 6.94 (1H, s), 9.28 (1H, s).
MS (ESI); m/z 217 (M+H)⁺

### Preparation example 63

### 4-(4-Chloro-3,5-dimethyl-1H-pyrazol-1-yl)-benzene-1,3-diol

200 mg of the title compound was afforded as a by-product during the silica gel column chromatography purification in Preparation example 58.
1H-NMR (CD₃OD); δ (ppm) 2.07 (3H, s), 2.20 (3H, s), 6.36 (1H, dd, J=2.4, 8.4Hz), 6.42 (1H, d, J=2.4Hz), 6.98 (1H, d, J=8.4Hz).
MS (ESI); m/z 239 (M+H)⁺

### 2. Preparation example of the topical liquid agent of the present invention

### Example 1

In the following, the present invention is explained by referring to Examples, but the present invention is not limited by these Examples. Note that the term, "Preparation example 44" in the followings means "Compounds of Preparation example 44".

| | |
|---|---|
| Preparation example 44 | 10g |
| Copolyvidone | 10g |
| Ethanol | 100 mL |

To a part of ethanol were dissolved Preparation example 44 and copolyvidone in this order, and made up the whole amount to 100 mL by ethanol to prepare a formulation.

Also, in view of the above-mentioned method, a topical liquid agent containing the following polymer in place of copolyvidone was prepared.

### 3. Formulation compatibility testing of the topical liquid agent of the present invention (confirmation of stability by remaining ratio)

Preparation example 44 (1 w/v%) and various kinds of additives (5 w/v%) were dissolved in ethanol (or an ethanol/ethyl acetate mixed solution (7:3)), the mixture was charged in a glass vial, and the plugged vials were stored at 40°C/75% RH for 3 months. As shown in Table 1, the remaining ratio of Preparation example 44 was 90% or more in all cases, and no additive which decreases the remaining ratio of Preparation example 44 was found.

**[Table 1]**

| Table 1: Compatibility test of Preparation example 44 and various additives | | |
|---|---|---|
| Sample | | Remaining ratio of Preparation example 44 |
| Viscosity-increasing | Copolyvidone | ≥90% |
| agent | Povidone | ≥90% |
| | Polyvinylacetal diethylaminoacetate | ≥90% |
| | Acrylic resin alkanolamine solution | ≥90% |
| | Hypromellose^{a)} | ≥90% |
| | Polyoxyethylene polyoxypropylene glycol^{a)} | ≥90% |
| | Aminoalkyl methacrylate copolymer RS | ≥90% |
| | Aminoalkyl methacrylate copolymer E | ≥90% |
| | Hydroxypropyl cellulose | ≥90% |
| Plasticizer | Propylene carbonate | ≥90% |
| | Propylene glycol | ≥90% |
| | Triacetine | ≥90% |
| | Triethyl citrate | ≥90% |
| | Macrogol 400 | ≥90% |
| | Diisopropyl adipate | ≥90% |
| Permeation enhancer | N-Acetyl-L-cysteine | ≥90% |

| | | |
|---|---|---|
| a) Ethanol/ethyl acetate mixed solution was used as a solvent. | | |

### 4. Confirmation of drug re-crystallization-preventing effect of the topical liquid agent of the present invention

To prevent drug re-crystallization on a nail when the topical liquid agent of the present invention was applied on the nail, compatibility of the drug and the polymer was evaluated by using differential scanning calorimetry (DSC). The polymer and Preparation example 44 were dissolved in ethanol with the respective mixing ratios, and 50 µL of each solution was dropped in a DSC aluminum pan and dried at 60°C for 1 hour. The dried material (film) was measured by the differential scanning calorimetry (DSC measurement conditions: crimped pan, measured range: 40°C to 120°C, heating rate is 3°C/min in the case of powder of Preparation example 44, and 5°C/min for the other samples, n=1). In the respective samples, enthalpy of the melting peak of Preparation example 44 at around 105°C was calculated, and compared to the melting enthalpy of Preparation example 44 when the powder alone was measured.

As shown in Table 2, in all of the polymers, accompanied with increase in an added amount of the polymer to the amount of the drug, the results showed the tendency that an amount of the drug existing in the film in a crystalline state was lowered.
[Table 2]

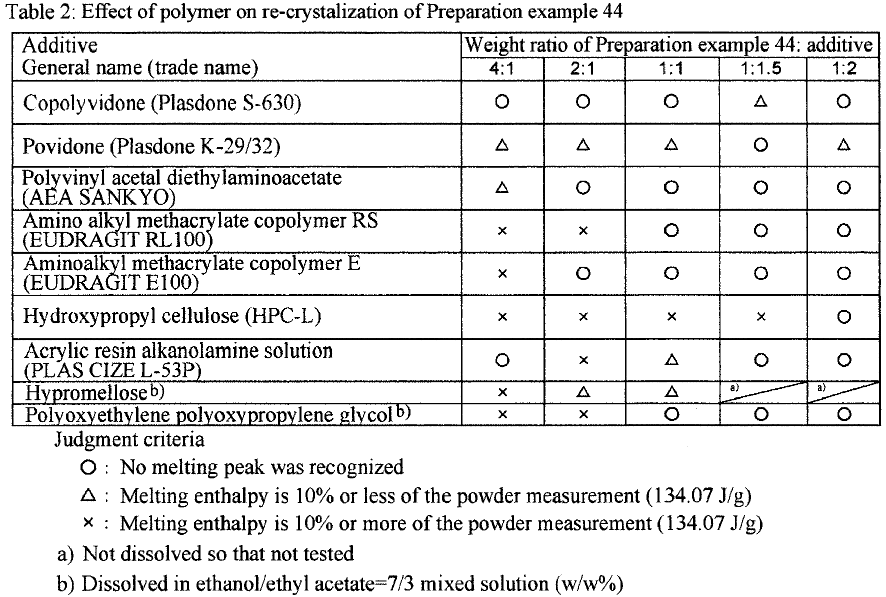

### 5. Evaluation of nail permeability of the topical liquid agent of the present invention

Nail permeability of the topical liquid agent of the present invention was examined by using a bovine hoof.

### 1) Preparation of test formulations

[Table 3]

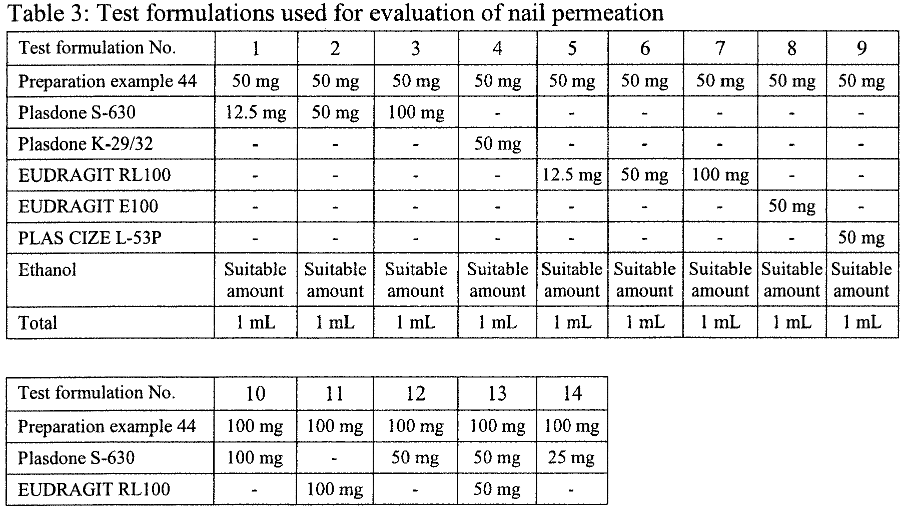

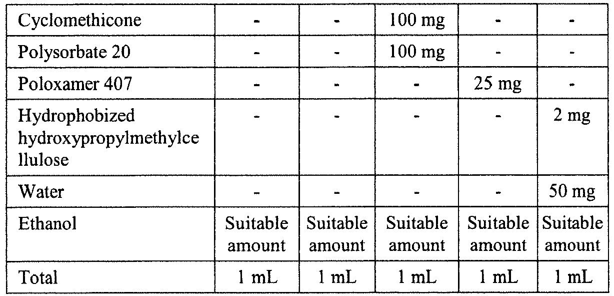

### 2) Preparation of bovine hoof slice

A bovine hoof having a thickness of 0.1 mm was cut to a suitable size, and dipped in ethanol for sterilization. After drying, a cylindrical plastic (height: about 2.5 mm, width: about 5 mm, thickness: about 1 mm) was mounted on the bovine hoof, fixed with an adhesive, allowed to stand overnight to completely adhere thereto.

### 3) Method

1% low melting point agarose was autoclaved, and apportioned into a petri dish with each 80 mL and solidified. A circular cylinder form (height: about 1.5 cm, diameter: about 2 cm, and volume: about 5 mL) of agarose piece was cut out from there with a cork hole borer made of a metal, and a bovine hoof to which a plastic is fixed was mounted on the agarose piece. 1 µL of the prepared test formulation was applied into a cylindrical plastic on the bovine hoof, covered with parafilm, allowed to stand at 28°C for 7 days, and then, the bovine hoof was removed. To the low melting point agarose was added 35 mL of distilled water, the gel was dissolved at 70°C, and the solution was quantitated by LC-MS to determine a nail permeated amount of Preparation example 44.

### 4) Results

As shown in Table 4, high nail permeability of Preparation example 44 could be recognized in all test formulations.
[Table 4]

**Table 4: Test results of nail permeability of Preparation example 44**

| Test formulation No. | Polymer | Polymer concentration | Nail permeated amount (µg) | Nail permeation ratio (%) |
|---|---|---|---|---|
| 1 | Plasdone S-630 | 1.25% w/v | 9.9 | 19.7 |
| 2 | Plasdone S-630 | 5% w/v | 12.8 | 25.7 |
| 3 | Plasdone S-630 | 10% w/v | 14.1 | 28.3 |
| 4 | Plasdone K-29/32 | 5% w/v | 13.3 | 26.7 |
| 5 | EUDRAGIT RL100 | 1.25% w/v | 11.4 | 22.8 |
| 6 | EUDRAGIT RL100 | 5% w/v | 12.2 | 24.4 |
| 7 | EUDRAGIT RL100 | 10% w/v | 9.4 | 18.9 |
| 8 | EUDRAGIT E100 | 5% w/v | 8.9 | 17.7 |
| 9 | PLAS CIZE L-53P | 5% w/v | 15.6 | 31.2 |
| 10 | Plasdone S-630 | 10% w/v | 30.2 | 30.2 |
| 11 | EUDRAGIT RL100 | 10% w/v | 32.7 | 32.7 |
| 12 | Plasdone S-630 | 5% w/v | 44.5 | 44.5 |
| | Cyclomethicone | 10% w/v | | |
| | Polysorbate 20 | 10% w/v | | |
| 13 | Plasdone S-630 | 5% w/v | 32.8 | 32.8 |
| | Poloxamer 407 | 2.5% w/v | | |
| 14 | Plasdone S-630 | 2.5% w/v | 38.2 | 38.2 |
| | Hydrophobized hydroxypropylmethylcellulose | 0.2% w/v | | |

### 6. Evaluation of dryness of the topical liquid agent of the present invention

**[Table 5]**

| Table 5: Film dried state and film appearance | | | | | | |
|---|---|---|---|---|---|---|
| Additive | Waster-soluble or water-insoluble | Dryness^{a)} | | | Appearance of film^{b)} | Tackiness of film ^{b)} |
| General name (trade name) | | Polymer concentration (mg/mL) | | | | |
| | | 50 | 100 | 150 | | |
| Copolyvidone (Plasdone S-630) | Water-soluble | ⊚ | ⊚ | ○ | Colorless transparent | None |
| Povidone (Plasdone K-29/32) | Water-soluble | ⊚ | ⊚ | ○ | Colorless transparent | None |
| Polyvinyl acetal diethylaminoacetate (AEA SANKYO) | Water-soluble | × | × | × | Slightly yellow transparent | None |
| Hydroxypropyl cellulose (HPC-L) | Water-soluble | × | × | Not carried out^{c)} | Colorless transparent | None |
| Acrylic resin alkanolamine solution (PLAS SIZE L-53P) | Water-soluble | ⊚ | Δ | × | Colorless transparent | None |
| Amino alkyl methacrylate copolymer RS (EUDRAGIT RL100) | Water-insoluble | ○ | Δ | × | Colorless transparent | None |
| Aminoalkyl methacrylate copolymer E (EUDRAGIT E100) | Water-insoluble | ○ | Δ | × | Colorless transparent | None |

| | | | | | | |
|---|---|---|---|---|---|---|
| a) An ethanol solution in which the polymer concentration was made 50 to 150 mg/mL was dropped to a glass plate, and the state thereof after 5 minutes was evaluated by a sensory test, and classified by the following standard. ⊚ : dried, ○ : surface was dried, Δ : tacky, × : liquid state b) Allowing to stand for 2 days, appearance and the presence or absence of tackiness of the remaining film was evaluated by a sensory test. c) Viscosity of the prepared liquid is high, so that it is judged not suitable for the test. | | | | | | |

### 7. Evaluation of formulations by sensory test

Test formulations shown in Table 6 were applied to nail and evaluated by sensory test of usability. The sensory test was performed for comprehensive usability in view of stickiness, fast-dry and appearance of the film (existence or non-existence of drug re-crystallization).

**Table 6 Test formulations used for the sensory test**

| Test formulation No. | 1 | 2 | 3 | 4 | 5 | 6 |
|---|---|---|---|---|---|---|
| Preparation example 44 | 100 mg | 100 mg | 100 mg | 100 mg | 100 mg | 100 mg |
| Copolyvidone | 100 mg | 50 mg | 25 mg | 50 mg | 50 mg | 25 mg |
| Cyclomethicone | - | - | - | 100 mg | - | - |
| Polysorbate 20 | - | - | - | 100 mg | - | - |
| Poloxamer 407 | - | - | - | - | 25 mg | - |
| Hydrophobized hydroxypropylmethylcellulose | - | - | - | - | - | 2 mg |
| Water | - | - | - | - | - | 50 mg |
| Ethanol | Suitable amount | Suitable amount | Suitable amount | Suitable amount | Suitable amount | Suitable amount |
| Total | 1 mL | 1 mL | 1 mL | 1 mL | 1 mL | 1 mL |
| Results | ○ | ○ | ⊚ | ⊚ | ○ | Δ |

| | | | | | | |
|---|---|---|---|---|---|---|
| ⊚ : very good, ○:good, Δ: not so good, ×: bad | | | | | | |

### 7. Antifungal activity against dermatophytes test

Measurement of the antifungal activity against dermatophytes of the compound of the formula (I) contained in the topical liquid agent of the present invention was carried out by the following method. The compound to be evaluated was used by dissolving in dimethyl sulfoxide (DMSO). As a medium for the test, RPMI1640 medium containing 0.165M of 3-morpholinopropanesulfonic acid (MOPS) was used. As fungi to be tested, *Trichophyton mentagrophytes* (*T. mentagrophytes)* ATCC18748 or *Trichophyton rubrum* (*T. rubrum)* ATCC10218 was used. The testing fungal strain was apportioned to 100 µl with a concentration of 1 × 10⁴ conidia/ml, mixed with a compound to be evaluated on a 96-well half area plate so that the DMSO concentration became 1%, and cultured at a cultivation temperature of 28°C for 3 days (*T. mentagrophytes)* or 4 days (*T. rubrum).* Thereafter, 5 µl of Cell Counting Kit 8 (WST8) was added thereto, and absorbances at 450 nm and 595 nm were measured to use these as background values. Thereafter, these were cultivated at 28°C for 5 hours (*T. mentagrophytes)* or overnight (*T. rubrum)* to cause a color, and absorbances at 450 nm and 595 nm were measured again. A growth inhibiting rate was calculated from the difference to the background values, and a 80% growth inhibiting concentration was made an MIC value (µg/ml).
[Table 7]

**Table 7: Antifungal activity against dermatophytes**

| Preparation example No. | MIC (*T. mentagrophytes)* | MIC (*T. rubrum)* | Preparation example No. | MIC (*T. mentagrophytes)* | MIC (*T. rubrum)* |
|---|---|---|---|---|---|
| 1 | B | B | 35 | C | C |
| 5 | C | D | 36 | E | D |
| 6 | C | E | 37 | B | B |
| 7 | C | E | 38 | C | B |
| 8 | C | E | 39 | C | C |
| 9 | A | A | 40 | E | D |
| 10 | D | E | 41 | D | D |
| 11 | C | D | 42 | C | D |
| 12 | C | E | 43 | E | D |
| 13 | A | B | 44 | A | A |
| 14 | B | B | 45 | A | A |
| 15 | C | C | 46 | E | D |
| 16 | C | E | 47 | B | B |
| 17 | A | A | 48 | B | B |
| 18 | B | B | 49 | E | D |
| 19 | C | C | 50 | B | B |
| 20 | C | D | 51 | E | D |
| 21 | A | B | 52 | A | A |
| 22 | C | D | 53 | B | B |
| 23 | C | D | 54 | A | A |
| 24 | A | C | 55 | A | A |
| 25 | B | B | 56 | A | A |
| 26 | A | A | 57 | B | B |
| 28 | B | C | 58 | B | A |
| 30 | A | A | 59 | B | B |
| 31 | B | B | 61 | C | C |
| 32 | B | B | 63 | A | A |
| 33 | B | A | Amorolfine hydrochloride | A | A |
| 34 | B | B | Terbinafine hydrochloride | A | A |
| MIC | | | MIC | | |
| (*T. mentagrophytes)* | | | (*T. rubrum)* | | |
| A: ≦1 µg/ml | | | A: ≦2 µg/ml | | |
| B: 2 to 8 µg/ml | | | B: 4 to 16 µg/ml | | |
| C: 16 to 32 µg/ml | | | C: 16 to 32 µg/ml | | |
| D: Inhibited 50% or more and less than 80% with 32 µg/ml | | | D: >32 to 64 µg/ml | | |
| E: Inhibited less than 50% with 32 µg/ml | | | E: >64 µg/ml | | |

### 9. Anti-Trichophyton activity of topical liquid agent

Permeability and antifungal activity against dermatophytes of the topical liquid agent of the present invention to the human nail were measured as follows by using a TurChub model available from MedPharm Co., Ltd.

A formulation to be tested was firstly prepared. The formulation to be tested is a topical liquid agent of Preparation example 44. The concentration of Preparation example 44 was made 0% (placebo), 2%, 5%, 10% and 15%, and copolyvidone (10%) and ethanol were added thereto to prepare the formulations.

The test formulation was applied onto human nail at the upper portion of a TurChub cell with an amount of 2 µl, and then, the TurChub cell was cultured between 20°C and 25°C for 7 days. Thereafter, a length of an inhibition zone against growth of dermatophyte (*Trichophyton rubrum)* inoculated on the agar in a receiver at the bottom of the TurChub cell was measured, and the antifungal activity against dermatophytes of the test formulation permeated to the human nail was confirmed.

As a result, the topical liquid agents for Preparation example 44 formed inhibition zones with sizes depending on the concentrations (2%, 5%, 10% and 15%) of the active substance. On the other hand, when the clinical formulation of ciclopirox or the clinical formulation of amorolfine was applied, no inhibition zone was formed.

According to the above, it could be understood that the topical liquid agent of the present invention permeated to the human nail, and showed the antifungal activity against dermatophytes concentration-dependently.
[Table 8]

**Table 8 Antifungal activity against dermatophytes**

| Test Formulation | Length (cm) of inhibition zone |
|---|---|
| Preparation example 44 liquid, 0% (placebo) | 0.00 |
| Preparation example 44 liquid, 2% | 1.23 |
| Preparation example 44 liquid, 5% | 1.78 |
| Preparation example 44 liquid, 10% | 2.48 |
| Preparation example 44 liquid, 15% | 3.00 |
| Amorolfine clinical formulation (Loceryl (Registered trade mark)) | 0.00 |
| Ciclopirox clinical formulation (Penlac (Registered trade mark)) | 0.00 |

### 10. Antifungal activity against dermatophytes of the topical liquid agent

Permeability and antifungal activity against dermatophytes of the topical liquid agent of the present invention to the bovine hoof were measured as follows.

After applying 2 µl of the test formulation into a cylindrical plastic on the bovine hoof, prepared in section 5.2), having a thickness of 0.1 mm, covered with parafilm, the bovine hoof was placed on agar medium inoculated with *Trichophyton mentagrophytes* (*T. mentagrophytes)* ATCC18748 as the fungus to be tested, and then the *Trichophyton mentagrophytes* was cultured at 28°C for 4 days. Thereafter, a length of an inhibition zone against growth of the *Trichophyton* was measured, and the antifungal activity against dermatophytes of the test formulation permeated to the bovine hoof was confirmed.

**Table 9**

| Test formulation No. | Polymer | Polymer concentration | Inhibition zone (cm) |
|---|---|---|---|
| 10 | Plasdone S-630 | 10% w/v | 5.27 |
| 11 | EUDRAGIT RL100 | 10% w/v | 5.03 |
| 12 | Plasdone S-630 | 5% w/v | 5.45 |
| | Cyclomethicone | 10% w/v | |
| | Polysorbate 20 | 10% w/v | |
| 13 | Plasdone S-630 | 5% w/v | 5.05 |
| | Poloxamer 407 | 2.5% w/v | |
| 14 | Plasdone S-630 | 2.5% w/v | 5.11 |
| | Hydrophobized hydroxypropylmethylcellulose | 0.2% w/v | |

### Results

From the above-mentioned evaluation results of 2 to 10, it could be confirmed that the topical liquid agent containing the compound of the formula (I) which is an active ingredient and a polymer easily formed a film by coating onto a skin, etc., had high antifungal activity against dermatophytes, high stability, nail permeability and re-crystallization-preventing effect of the active ingredient. In addition, it was rapid-dry, and as the polymer as an additive, it was shown that both of the water-soluble polymer and the water-insoluble polymer were preferred. Above all, copolyvidone, povidone, an acrylic resin alkanolamine and an aminoalkyl methacrylate copolymer are preferred. Further, addition of a viscosity-increasing agent is preferable, in particular, silicone derivatives.

### UTILIZABILITY IN INDUSTRY

According to the present invention, a topical liquid agent for nail and/or skin having antifungal activity against dermatophytes, and having higher nail permeability can be provided.

## Claims

1. A topical liquid agent for nail and/or skin for prevention or treatment of dermatophytosis which comprises a film-forming agent and a compound represented by the formula: 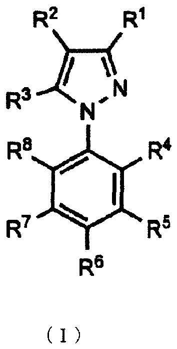 wherein
R¹ represents a hydrogen atom, C₁₋₆ alkyl, or trifluoromethyl,
R² represents a hydrogen atom, C₁₋₆ alkyl, halogen, -COO(C₁₋₆ alkyl), or (CH₂)₁₋₃COOR where R represents a hydrogen atom or C₁₋₆ alkyl,
R³ represents a hydrogen atom, C₁₋₆ alkyl, amino, trifluoromethyl, or OR where R represents a hydrogen atom or C₁₋₆ alkyl,
R⁴ represents a hydroxyl group,
R⁵ represents a hydrogen atom, C₁₋₆ alkyl, a hydroxyl group, or halogen,
R⁶ represents a hydrogen atom, C₁₋₆ alkyl, trifluoromethyl, halogen, amino, -NR^{a}R^{b}, nitro, hydroxy-C₁₋₆ alkyl, -CONR^{a}R^{b}, -COO(C₁₋₆ alkyl), -COOH, -(CH₂)₁₋₃COOR, or OR^{a} where R represents a hydrogen atom or C₁₋₆ alkyl, R^{a} and R^{b} may be the same or different from each other, and each represent a hydrogen atom, C₁₋₆ alkyl, or C₁₋₆ acyl,
R⁷ represents a hydrogen atom, C₁₋₆ alkyl, -OR where R represents a hydrogen atom or C₁₋₆ alkyl, or halogen,
R⁸ represents a hydrogen atom, C₁₋₆ alkyl, a hydroxyl group, amino, or nitro,
or a salt thereof.

2. The topical liquid agent for nail and/or skin according to Claim 1, which contains the compound represented by the formula (I) where
R¹ is a hydrogen atom or C₁₋₆ alkyl,
R² is a hydrogen atom, C₁₋₆ alkyl, halogen, or -(CH₂)₁₋₃COOR where R represents a hydrogen atom or C₁₋₆ alkyl,
R³ is a hydrogen atom, or C₁₋₆ alkyl,
R⁴ is a hydroxyl group,
R⁵ is a hydrogen atom,
R⁶ is a hydrogen atom, C₁₋₆ alkyl, trifluoromethyl, halogen, amino, -NR^{a}R^{b}, nitro, hydroxy-C₁₋₆ alkyl, -CONR^{a}R^{b}, -COOH, or OR^{a} where R^{a} and R^{b} may be the same or different from each other, and each represent a hydrogen atom, C₁₋₆ alkyl, or C₁₋₆ acyl,
R⁷ is a hydrogen atom, and
R⁸ is a hydrogen atom, C₁₋₆ alkyl, amino, or nitro,
or a salt thereof.

3. The topical liquid agent for nail and/or skin according to Claim 1, which contains the compound represented by the formula (I) where
R¹ is C₁₋₆ alkyl,
R² is a hydrogen atom, C₁₋₆ alkyl, or halogen,
R³ is C₁₋₆ alkyl,
R⁴ is a hydroxyl group,
R⁵ is a hydrogen atom,
R⁶ is a hydrogen atom, C₁₋₆ alkyl, halogen, or OR^{a} where R^{a} represents a hydrogen atom, C₁₋₆ alkyl, or C₁₋₆ acyl,
R⁷ is a hydrogen atom, and
R⁸ is a hydrogen atom,
or a salt thereof.

4. The topical liquid agent for nail and/or skin according to Claim 1, which contains the compound represented by the formula (I) where R¹ is C₁₋₆ alkyl, R² is a hydrogen atom, R³ is C₁₋₆ alkyl, R⁴ is a hydroxyl group, R⁵ is a hydrogen atom, R⁶ is C₁₋₆ alkyl, R⁷ is a hydrogen atom, R⁸ is a hydrogen atom,
or a salt thereof.

5. The topical liquid agent for nail and/or skin according to Claim 1, which contains 2-(3,5-dimethyl-1H-pyrazol-1-yl)-5-methylphenol.

6. The topical liquid agent for nail and/or skin according to any one of Claims 1 to 5, wherein the film-forming agent contains a polymer.

7. The topical liquid agent for nail and/or skin according to Claim 6, wherein the polymer is copolyvidone, povidone, an acrylic resin alkanolamine, or an aminoalkyl methacrylate copolymer.

8. The topical liquid agent for nail and/or skin according to any one of Claims 1 to 7, the liquid agent comprising a lower alcohol.

9. The topical liquid agent for nail and/or skin according to any one of claims 1 to 8, the liquid agent comprising a viscosity-increasing agent.

10. The topical liquid agent for nail and/or skin according to claim 9, wherein the viscosity-increasing agent is a silicone derivative, hydrophobized hydroxypropylmethylcellulose or poloxamer.

11. The topical liquid agent for nail and/or skin according to any one of Claims 1 to 10, wherein the compound represented by the formula (I) or a salt thereof is contained in an amount of 1 to 30% by mass based on the whole amount of the liquid agent.

12. The topical liquid agent for nail and/or skin according to any one of Claims 1 to 10, wherein the compound represented by the formula (I) or a salt thereof is contained in an amount of 1 to 20% by mass based on the whole amount of the liquid agent.

13. The topical liquid agent for nail and/or skin according to any one of Claims 1 to 10, wherein the compound represented by the formula (I) or a salt thereof is contained in an amount of 5 to 15% by mass based on the whole amount of the liquid agent.

14. The topical liquid agent for nail and/or skin according to any one of Claims 1 to 13, wherein it is used for prevention or treatment of tinea unguium.
